(19) Europäisches Patentamt
European Patent Office
Office européen des brevets

(11) **EP 2 744 427 B1**

(12) **EUROPEAN PATENT SPECIFICATION**

(45) Date of publication and mention
of the grant of the patent:
**05.06.2019 Bulletin 2019/23**

(51) Int Cl.:
***A61B 17/29*** *(2006.01)*     ***A61B 34/00*** *(2016.01)*
***A61B 34/30*** *(2016.01)*     *A61B 90/00* *(2016.01)*

(21) Application number: **12824278.1**

(22) Date of filing: **15.08.2012**

(86) International application number:
**PCT/US2012/050988**

(87) International publication number:
**WO 2013/025831 (21.02.2013 Gazette 2013/08)**

(54) **MEDICAL INSTRUMENT WITH FLEXIBLE JAW MECHANISMS**

MEDIZINISCHES INSTRUMENT MIT FLEXIBLEM SPANNBACKENMECHANISMUS

INSTRUMENT MÉDICAL AYANT DES MÉCANISMES DE MÂCHOIRE FLEXIBLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.08.2011 US 201113210142
15.08.2011 US 201113210196**

(43) Date of publication of application:
**25.06.2014 Bulletin 2014/26**

(73) Proprietor: **Intuitive Surgical Operations, Inc.
Sunnyvale, CA 94086 (US)**

(72) Inventor: **BLUMENKRANZ, Stephen J.
Los Altos Hills, California 94022-4540 (US)**

(74) Representative: **MacDougall, Alan John Shaw et al
Mathys & Squire LLP
The Shard
32 London Bridge Street
London SE1 9SG (GB)**

(56) References cited:
WO-A1-00/01304     DE-A1- 19 537 320
DE-B3-102007 030 856     US-A- 5 052 402
US-A- 5 906 630     US-A- 6 149 607
US-A1- 2003 036 748     US-A1- 2004 260 198
US-A1- 2009 198 272     US-A1- 2010 030 238



## Description

### TECHNICAL FIELD

The present invention relates generally to a medical instrument, and more particularly to a medical instrument with jaws and/or to a medical instrument component for holding a mechanism attached therein in different positions and orientations.

### BACKGROUND

Modern tools and manipulating instruments, especially instruments with jaws for performing surgical operations, such as cutting, grasping and holding, are providing increasing levels of functionality and strength to support modem needs including applications in minimally invasive and micro-surgery. It is desirable to further reduce the diameter of these instruments to reduce incision size and post-operative pain and scarring and to address smaller anatomy in pediatric, vascular and nerve surgery, and in micro-surgery such as ophthalmic surgery. However, the mechanisms available for positioning and orienting the jaws of smaller manipulating instruments are not efficient and often lack precision.

Often, the tools available are not efficient in applying the correct amount of force precisely or being precisely positioned. As surgical instruments decrease in size, a number of problems occur with mechanisms having jaws, such as forceps, graspers, and scissors.

New surgical techniques have created less invasive procedures, such as minimally invasive surgery (MIS) and robotic MIS, which has created the need for smaller diameter instruments. The need for small instruments is motivated by patient concern for cosmetic healing with minimal or no scars and less incision size related post-operative pain.

The development of less invasive medical instruments is also motivated by surgeons who need smaller instruments to address smaller anatomy such as small blood vessel and nerve re-anastomosis, ophthalmic surgery, vasectomy reversal and the like. Another surgeon motivation for developing less invasive medical instruments and procedures is the desire to make patients happy with less noticeable scarring, less post-operative pain and more rapid healing.

One of the technical obstacles to producing these less invasive medical instruments is the transmission of force from the mechanical actuator to the instrument jaw or end effector on the other end. The delivery of too much force or too little can present a surgeon with additional unwanted complications in surgery.

Another difficulty is the precise positioning and movement of the medical instrument jaws or end effector. Providing a precise control through a system of linkages can be difficult. The combined linkages have inherent movement error called "hysteresis", which is usefully thought of as lost motion or wasted energy. The hysteresis of a medical instrument is caused by the friction between moving parts, and the stretching of interconnecting parts.

Block and tackle style mechanisms for jaw actuation can provide greater mechanical advantage to the actuating cable but add to parts count, assembly cost, and mechanism friction.

In cable actuated hinge mechanisms using pins or shafts on which portions of the hinge pivot, the cable force increases the pivot pin friction resisting hinge rotation. The pivot pin friction will resist movement until sufficient actuating force is applied to overcome the friction. This means greater actuating force than desired must be applied in order to initiate hinge rotation. This undesirable situation can cause excessive motion as the friction force reduces dramatically once motion is initiated.

Since the hysteresis, usefully thought of as lost motion or wasted energy, of any mechanism varies with the product of the mechanism friction multiplied by its drive train compliance, the combined effect of these friction and compliance increases is a large increase in wrist motion hysteresis as the cross-sectional diameter of an instrument, such as a gripper, decreases for a given type of hinge mechanism. This is particularly detrimental when there is rubbing friction with higher starting friction that results in uneven or unpredictable motion effects sometimes called stiction. Therefore, in order to enable smaller smoothly functioning surgical instrument wrists, it is also desirable to have a new wrist mechanism with lower friction.

The need to reduce costs, to improve efficiencies and performance, and to meet competitive pressures adds an even greater urgency to the critical necessity for finding answers to these problems. Solutions to these problems have been long sought but prior developments have not taught or suggested any solutions and, thus, solutions to these problems have long eluded those skilled in the art.

DE 195 37 320 discloses a system for operating the handling unit which has at least one movable handling part with first and second holding parts, connected respectively across first and second flexible connecting parts of the joint connection, with the movement of the first holding part to the second holding part or vice versa. With the bringing back of the part moved relatively to the other part reverses in its original position. Passive auxiliary functions can be integrated with a corresponding design of the components, e.g., ducts can be provided for the reception of laser and light conductors also fibre optics. Ducts also for controlling the handling unit and cable ducts for the supply of a coagulating HF current.

SUMMARY

Some embodiments of the present invention provide a medical instrument as set out in the appended claims. The medical instrument comprising a unitary jaw structure having a connector portion with a width, a first jaw portion flexibly integral with the connector portion at one side of the width, a second jaw portion integral with the connector portion at the other side of the width, a first arm portion integral with the first jaw portion and extending from the first jaw portion to the other side of the width, and an actuator portion flexibly integral with the first arm portion at the other side of the width for causing rotating motion of the first jaw portion upon linear motion of the actuator portion, the actuator portion extending centrally through the connector portion, wherein the connector portion, the first jaw portion, the second jaw portion, the first arm portion, and the actuator portion are all formed in a single piece.

Some examples of the present disclosure further provide a medical instrument that includes: a unitary wrist structure having a first connector portion having a lower stop surface, a compact flexure integral with the first connector portion, and a second connector portion integral with the compact flexure with the second connector portion having an upper stop surface integral with the compact flexure below the lower stop surface and forming an angle with the lower stop surface.

Certain examples of the disclosure have other steps or elements in addition to or in place of those mentioned above. The steps or elements will become apparent to those skilled in the art from a reading of the following detailed description when taken with reference to the accompanying drawings.

BRIEF DESCRIPTION OF THE DRAWINGS

FIG. 1 is a medical instrument with a unitary jaw structure in an embodiment of the present invention.

FIG. 2 is an enlarged detailed isometric view of the unitary jaw structure.

FIG. 3 is a detailed cut-away isometric view of the unitary jaw structure.

FIG. 4 is an exemplary view of the unitary jaw structure opening.

FIG. 5 is an exemplary view of the unitary jaw structure closing.

FIG. 6 is an enlarged detailed isometric view of the unitary wrist.

FIG. 7 is an enlarged front view of the unitary wrist structure.

FIG. 8 is an enlarged side view of the unitary wrist structure.

FIG. 9 is an enlarged side view of the unitary wrist structure partially flexed forward.

FIG. 10 is an enlarged side view of the unitary wrist structure partially flexed forward and partially flexed to the left.

FIG. 11 is an enlarged detailed isometric view of another unitary joint structure.

FIG. 12 is a front view of the unitary joint structure.

FIG. 13 is a front view of the unitary joint structure in a fully flexed position.

FIG. 14 is a side view of the unitary joint structure.

FIG. 15 is an enlarged detailed isometric view of another unitary wrist structure.

FIG. 16 is an enlarged front view of the unitary wrist structure.

FIG. 17 is an enlarged side view of the unitary wrist structure.

FIG. 18 is an enlarged side view of the unitary wrist structure partially flexed forward.

FIG. 19 is an enlarged side view of the unitary wrist structure partially flexed forward and partially flexed to the right.

FIG. 20 is an enlarged isometric view of a compact flexure structure.

FIG. 21 illustrates an embodiment of the unitary jaw structure mounted to an example of the unitary wrist structure.

DETAILED DESCRIPTION

**UNITARY JAW MECHANISM**

The following embodiments are described in sufficient detail to enable those skilled in the art to make and use the invention. It is to be understood that other embodiments would be evident based on the present disclosure, and that system, process, or mechanical changes may be made without departing from the scope of the present invention.

In the following description, numerous specific details are given to provide a thorough understanding of the invention. However, it will be apparent that the invention may be practiced without these specific details. In order to avoid obscuring the present invention, some well-known devices, instrument configurations, and process steps are not disclosed in detail.

For expository purposes, the term "horizontal" as used herein is defined as the horizontal direction seen when viewing the drawing as indicated by the figure designation of "FIG.". The term "vertical" refers to a direction perpendicular to the horizontal as just defined. Terms, such as "above", "below", "bottom", "top", "side" (as in "sidewall"), "higher", "lower", "upper", "over", and "under", are defined with respect to the horizontal, as shown in the figures. The term "on" means there is direct contact between the elements described. Generally, the invention can be operated in any orientation.

Also, in the following description, connected and coupled are used to describe a relationship between two members. The term "connected" means that the two members are physically and directly joined to each other.

Different members can be connected in variety of ways. For example, different members can be connected by being formed adjacent to each other, such as through molding or carving. Also, for example, different members can be connected by being attached together, such as through adhesives, fasteners, welds, or brazing. The term "wrist" means a structure capable of two degrees of freedom, by being able to bend in more than one direction concurrently. The term "joint" means a structure that is capable one degree of freedom, by being able to bend in two directions that are 180 degrees apart.

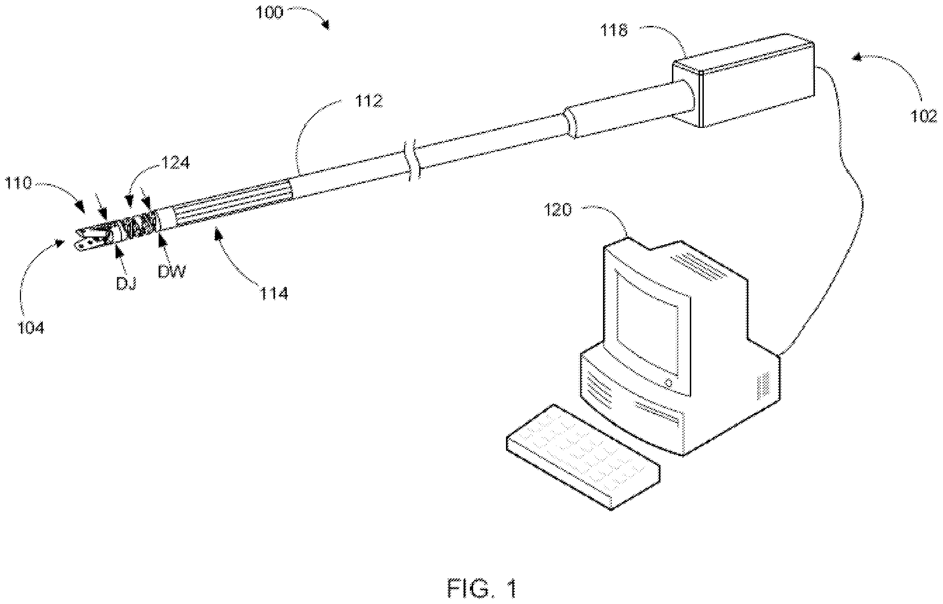

The term "coupled" means that the two members are physically linked through one or more other members. The phrases "reciprocating motion" and "reciprocating movement" are defined to describe a repetitive up-and-down or back-and-forth linear or angular rotational motion. The phrases "distal" and "proximal" are defined to respectively indicate the directions designated by the related arrows in FIG. 1 or along the path of connectivity between the point where the instrument couples to the robot arm (proximal) and the instrument tip that contacts surgical patient tissue (distal).

The drawings showing embodiments of the system are semi-diagrammatic and not to scale and, particularly, some of the dimensions are for the clarity of presentation and are shown exaggerated in the drawing FIGs. Similarly, although the views in the drawings for ease of description generally show similar orientations, this depiction in the FIGs. is arbitrary for the most part. Generally, the invention can be operated in any orientation.

Often, the surgical instruments available are not efficient in applying the correct amount of jaw grip force or positioning the jaws precisely. As surgical instruments decrease in size, a number of problems occur with mechanisms having jaws, such as forceps, graspers, and scissors.

As the ratio of jaw length to jaw actuating lever length increases, the mechanical advantage of an actuating system decreases. Therefore, for a desired jaw closure force, most actuating system cables and pulleys require a relatively greater force to be exerted by the actuating cable as surgical instrument diameter decreases.

In jaw systems using pins or shafts on which the individual jaws pivot, the greater cable force increases the pivot pin friction resisting jaw rotation. This means greater actuating force than desired must be applied for jaw actuation.

The higher cable force also requires a corresponding increase in cross-sectional shear area of the pivot pin supporting the jaws thus requiring a larger pivot pin diameter to prevent failures. The ratio of actuator pulley diameter to pivot pin diameter decreases as a result of the decrease in pulley size and increase in pin size, which further increases the frictional torque resisting jaw rotation and further increases the actuating cable force to achieve a given jaw force.

The higher frictional forces and lower mechanical advantage increase cable axial deflection or stretch so that greater movement than desired of the proximal end of the actuating cable is required for a predetermined amount of jaw rotation and force, which means the effective drive train compliance is increased.

Since the hysteresis of any mechanism varies with the product of the mechanism friction multiplied by its drive train compliance, the combined effect of these friction and compliance increases is a large increase in hysteresis as the cross-sectional diameter of an instrument, such as a gripper, decreases for a given type of jaw mechanism. This is particularly detrimental when there is rubbing friction with higher starting friction that results in uneven or unpredictable motion effects sometimes called stiction or stick-slip.

Therefore, in order to enable smaller smoothly functioning surgical instruments, it is desirable to have a new jaw mechanism with lower friction, greater jaw lever mechanical advantage, and lower effective drive train compliance.

Others have addressed these problems by providing heavy solid actuating rods with higher axial stiffness (lower compliance). However, large solid jaw actuating rods interfere with the flexibility or prevent the use of an articulated wrist used to position and orient the jaws and also may be susceptible to bending stress fatigue failure in high cycle life uses. Thus heavy solid rod actuating members are best adapted to manual laparoscopic surgical instruments without a wrist and poorly adapted to robotic laparoscopic surgical instruments with an articulated wrist.

Referring now to FIG. 1, therein is shown a medical instrument 100 with a unitary jaw structure 110 in an embodiment of the present invention. The medical instrument 100 has a proximal end 102 and a distal end 104.

The medical instrument 100 can include the unitary jaw structure 110 at the distal end 104, a tube 112 with actuating members 114, and an actuator system 118 at the proximal end 102. The unitary jaw structure 110 in an embodiment of the present invention is analogous to a human hand. The term "unitary" means a structure of a single unit of material. The unitary jaw structure 110 can be manufactured from a single material as a single unit.

The medical instrument 100 can include a wrist mechanism 124, analogous to a human wrist, attached to or integrally formed with the unitary jaw structure 110. The wrist mechanism 124 can be connected to the unitary jaw structure 110 using an adhesive or a mechanical fastener or it may also be welded or brazed. In an alternative embodiment, the unitary jaw structure 110 and the wrist mechanism 124 can together be manufactured from a single material as a single unit.

The unitary jaw structure 110 can be carved or shaped out of a single unit of material, such as plastic or metal alloy. For examples, the unitary jaw structure 110 can be formed by cutting and carving polypropylene plastic or metal alloy or can be formed by using wire electrical discharge machining (EDM) process and post treatment to remove surface layer re-melt, such as when used to shape Nitinol alloy.

The unitary jaw structure 110 can also be molded into shape. For example, the unitary jaw structure 110 can be molded plastic or cast metal. The unitary jaw structure 110 also can be formed as a single injection molding of a polymer, such as fiber reinforced polyether ether ketone (PEEK), or by metal injection molding (MIM) into a die or mold that has a continuous cavity.

The unitary jaw structure 110 is shown having a cylindrical configuration with a taper narrowing towards the distal end 104. The taper allows the unitary jaw structure 110 to project into tight spaces and move between obstacles such as organs or blood vessels.

The unitary jaw structure 110 provides for improved manufacturing operations with the advantage of eliminating assembly operations because of its one piece structure. Because of reduced cost, it is feasible to make the unitary jaw structure 110 for single use applications. A single use tool avoids the need for the handling and processing associated with cleaning and resterilization after use, as well as the need for certain instrument design requirements for resterilization, such as the ability to withstand autoclaving.

The tube 112 holds the unitary jaw structure 110 and the unitary wrist structure 124 at a location in space. For example, the tube 112 can be straight tube of a medical instrument.

For illustrative purposes the tube 112 is shown as a hollow cylindrical member encasing the actuating members 114 within the tube 112. However, it is understood that the tube 112 can be different and have various cross-sectional shapes, or be solid and have external versions of the actuating members 114.

The unitary jaw structure 110 is attached to the unitary wrist structure 124 at the distal end 104 of the tube 112 and the actuator system 118 at the proximal end 102. Generally, the unitary jaw structure 110 is the more distal portion and the wrist mechanism 124 is the more proximal portion.

The wrist mechanism 124 is a member that bends or provides multi-axis movement to change the relative position and orientation of the unitary jaw structure 110. For example, the wrist mechanism 124 can be a ball and socket or may have more than one pin jointed hinges at right angles to each other.

Also, for example, the wrist mechanism 124 can be a flexible mechanism manufactured from a single material as a single unit that can move to allow for the medical instrument 100 to position the unitary jaw structure 110. The wrist mechanism 124 can be a flexible member that can bend without discrete pivoting pin joints. The wrist mechanism 124 is supported by the tube 112.

In one embodiment, the unitary jaw structure 110 has a diameter, depicted as DJ, and the wrist mechanism 124 has a diameter DW, as depicted in FIG. 1. In one embodiment, DW is equal to DJ so that both portions of the unitary jaw structure 110 can enter easily through the same cannula in an incision during a minimally invasive surgical procedure.

The actuator system 118 exerts forces coupled by the actuating members 114 to bend the wrist mechanism 124 and to actuate the unitary jaw structure 110. The actuating members 114, for example, can be a rod, cable, or cable and pulley system that is pushed or pulled to bend the wrist mechanism 124 along the direction of applied force. The actuator system 118 can also be coupled through the actuating members 114 to convey the forces to cause rotating reciprocation motion of the unitary jaw structure 110.

The actuator system 118 may include or may be coupled to electrical, hydraulic, or pneumatic power systems to generate the applied forces. A control system 120 can be coupled to the actuator system 118 for controlling the amount of applied forces and motion for the unitary jaw structure 110 and the wrist mechanism 124. The control system 120 is a mechanism that can control the operation of the unitary jaw structure 110. For example, the control system 120 can be a computer and motor assembly or an assembly of handles, gears, and levers.

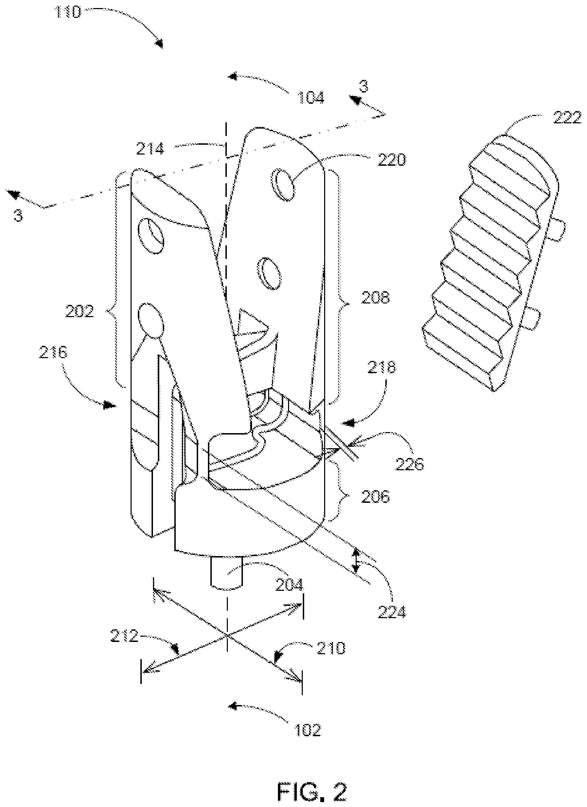

Referring now to FIG. 2, therein is shown an enlarged detailed isometric view of the unitary jaw structure 110. The unitary jaw structure 110 includes a first jaw portion 202, an actuator portion 204, a connector portion 206, and a second jaw portion 208.

The unitary jaw structure 110 has a first transverse dimension 210 and a second transverse dimension 212 along a plane orthogonal to a center line 214. The first transverse dimension 210 and the second transverse dimension 212 are shown to be the same but do not need to be and may be adjusted based on the geometry of the unitary jaw structure 110. In the case in which they are equal, the unitary jaw structure 110 may be circular in cross section as illustrated in FIG. 2.

The first jaw portion 202 is a portion of the unitary jaw structure 110 that is radially symmetric to the second jaw portion 208. The first jaw portion 202, the second jaw portion 208, or a combination thereof can be used to grab, manipulate, cut, or perform a combination of operations. The first jaw portion 202 and the second jaw portion 208 can rotate equal distances in opposite direction simultaneously along arcs centered on respectively a first flexible hinge 216 and a second flexible hinge 218.

The first jaw portion 202 can extend away from the connector portion 206. The first jaw portion 202 can have a variety of shapes. For example, the first jaw portion 202 can be trapezoidal, rectangular, or oval in shape extending away from the connector portion. Also, for example, the first jaw portion 202 can have a planar outer surface or a rounded outer surface.

The first flexible hinge 216 and the second flexible hinge 218 are flexible members, also known as flexures, that couple two other relatively rigid members. For example, the first flexible hinge 216 can couple the first jaw portion 202 and the connector portion 206. Also, for example, the second flexible hinge 218 can couple the second jaw portion 208 and the connector portion 206.

The first flexible hinge 216 and the second flexible hinge 218 can have a hinge length 224 and a hinge thickness 226. The hinge length 224 can be the length of the first flexible hinge 216 and the second flexible hinge 218, which can be measured in a direction parallel to the center line 214. The hinge thickness 226 can be the thickness of the first flexible hinge 216 and the second flexible hinge 218, which can be measured in a direction parallel to the second transverse dimension 212.

The hinge thickness 226 can be sufficiently less than the hinge length 224 that it provides a bending compliance between the coupled rigid members allowing one rigid member to rotate with respect to the other about an axis perpendicular to the hinge length 224 and the hinge thickness 226. The axis for rotation can be located at the midpoint of the hinge length 224 and the hinge thickness 226 when the flexure is straight.

The hinge length 224 and the hinge thickness 226 can have a ratio of length to thickness such that the bending strain and the resulting stress in the flexure are within limits based on its material properties, such as yield strength or fatigue strength limit, angular range of motion, and required flexing motion cycles. The first flexible hinge 216 and the second flexible hinge 218 may rigidly transmit forces along at least the hinge length 224 between the two coupled members. Advantageously, when moving, the first flexible hinge 216 and the second flexible hinge 218 have only low internal hysteresis losses in the material, also called equivalent friction, which are significantly lower than the corresponding actual friction losses in a similarly loaded pin jointed hinge. Hysteresis loss is the loss in motion or energy in mechanisms and structures due to the physical property thereof.

The first flexible hinge 216 and the second flexible hinge 218 can be a compact flexure hinge. A compact flexure hinge is a flexure made from a plastic material preferably such as injection molded polypropylene or ultra-high molecular weight polyethylene (UHMW-PE) whose material properties permit a short flexure length while at the same time permitting a high number of flexing motion cycles. For example, the flexure length can be less than twice the hinge thickness 226 or equivalent thickness, such as the thickness at the vertex point when the hinge has a concave shape. The compact flexure hinge can be bounded by concave curved surfaces.

The flex life of a compact flexure hinge, the number of times a compact flexure hinge can be reliably flexed or bent, can be enhanced by providing that the melted plastic flows through the hinge from one coupled rigid member toward the other as the mold fills and by flexing the hinge fully in both directions immediately upon removal from the mold.

Also advantageously, the compact flexure hinge may reduce cost by eliminating multiple separate components and associated assembly labor as well as by using a low cost material. The compact flexure hinge benefits the rigidity of the jaw hinge in the direction of the flexure thickness.

Because of their reduced length in relation its thickness, the first flexible hinge 216 and the second flexible hinge 218 when configured as the compact flexure hinges, advantageously provide relatively greater stiffness than conventional elongated flexures with respect to forces between the coupled members in the direction of the hinge thickness 226.

In addition to plastic or metal injection molding or cutting of plastic or metal by machining methods, including metal cutting by wire EDM, the first flexible hinge 216 and the second flexible hinge 218 permit exploitation of a planar photo-lithographic metal alloy plating process by orienting the flexible hinges in the plane of the plated layers to enable unitary structures of 1 mm diameter or smaller. Thus the invention enables manufacture of a functional medical instrument jaw of unprecedented smaller size and further enables surgery on correspondingly smaller anatomy such as re-anastomosis of small blood vessels and nerves or manipulation and repair of structures inside the eye.

The first jaw portion 202 can be flexibly integral with the connector portion 206 through the first flexible hinge 216. The second jaw portion 208 can also be flexibly integral with the connector portion 206 through the second flexible hinge 218.

For illustrative purposes, the first flexible hinge 216 and the second flexible hinge 218 have been shown as having the same shape and dimensions, and having a uniform thickness. However, it is understood that the two hinges can be different. For example, the two hinges can have different shapes, dimensions, or a combination thereof and the hinge thickness 226 can vary along the hinge length 224, along a direction perpendicular to the hinge length 224, or both. It is also understood that in an alternate embodiment one jaw portion could be stationary while the other has the reciprocating motion for gripping objects.

The first jaw portion 202 and the second jaw portion 208 can have an adaptor 220 for fastening a task adaptor 222 for specific tasks. The adaptor 220 is a hole or a fastening mechanism for attaching the task adaptor. For example, the adaptor 220 can be a hole or a mechanical fastener for accommodating, such as by fitting or fastening, the task adaptor 222 to the first jaw portion 202, the second jaw portion 208, or a combination thereof.

The task adaptor 222 can be a pad or mechanism for adapting the unitary jaw structure 110 for special tasks. For example, the task adaptor 222 can be a serrated pad for non-slip gripping or a pad with a semi-circular indentation for encircling and grabbing cylindrical objects, such as blood vessels.

Further, it is understood that the first jaw portion 202 and the second jaw portion 208 can be different. For example, the first jaw portion 202 and the second jaw portion 208 can be different from each other in shape. Also, for example, the first jaw portion 202 can be rectangular in shape with the first transverse dimension 210 constant throughout the first jaw portion 202 to provide a broader clamping surface.

The actuator portion 204 is the portion of the unitary jaw structure 110 that causes the reciprocating motion of one or both jaw portions. The actuator portion 204 can be a solid member, such as a bar or wire, that can be moved.

The actuator portion 204 can have rod or a column portion. One of the actuating members 114 of FIG. 1 can be connected or coupled to the rod or the column portion of the actuator portion 204, either without a wrist mechanism or through the wrist mechanism 124 of FIG. 1, to move the actuator portion 204 relative to the connector portion 206.

For example, pulling one of the actuating members 114 at the proximal end 102 of the medical instrument 100 can cause the first jaw portion 202 and the second jaw portion 208 to close. The release of the actuating members 114 where there is no pull or push forces on them can cause the unitary jaw structure 110 to open to a position where the first flexible hinge 216 and the second flexible hinge 218 are straight. Similarly, a push force may be applied to the actuator portion 204 through the actuating members 114 to force one or both jaw portions to open more fully if desired.

In alternative embodiment, the actuator portion 204 can be coupled only to the first jaw portion 202 so that the movement of the actuator portion 204 can cause rotating movement of only the first jaw portion 202.

The hinge portions can be resilient. Therefore, the release of the actuating members 114 where there is no pull or push force on the actuator portion 204 can cause the first jaw portion 202, the second jaw portion 208, or both to move to a position where the first flexible hinge 216 and the second flexible hinge 218 are at a defined neutral position, such as substantially parallel to one another.

The connector portion 206 can be connected to the distal part of the wrist mechanism 124 that is in turn connected to the tube 112. The connector portion 206 can also be directly connected to the tube 112 or can be coupled by an alternative structure.

In one aspect, the first flexible hinge 216 and the second flexible hinge 218 are low equivalent friction structures, such as resilient structures, for allowing movement of the first jaw portion 202 and the second jaw portion 208 relative to the connector portion 206.

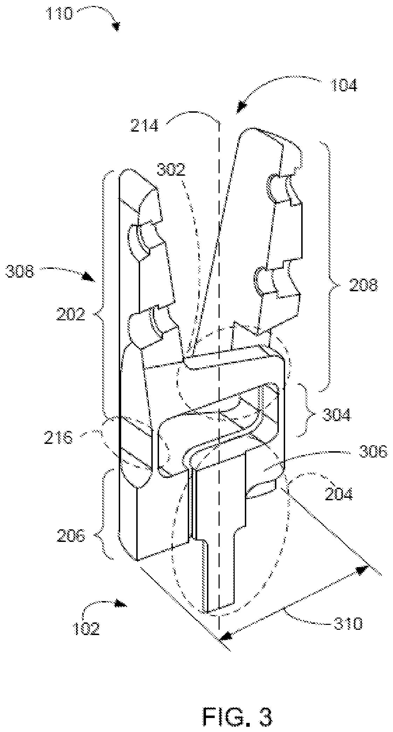

In an alternative embodiment, the first flexible hinge 216 and the second flexible hinge 218 can be the compact flexure hinges with all the attendant advantages. The section line 3--3 shows the location and direction of view of the cross-section of FIG. 3 below.

Referring now to FIG. 3, therein is shown a detailed cut-away isometric view of the unitary jaw structure 110. The unitary jaw structure 110 can include a flexible jaw mechanism 308 at the distal end 104 and the connector portion 206 at the proximal end 102. The flexible jaw mechanism 308 comprises the first jaw portion 202, the second jaw portion 208, and the actuator portion 204. The flexible jaw mechanism 308 includes a first arm portion 302 and a first arm hinge portion 304 integral with the first jaw portion 202 and the actuator portion 204 respectively. The actuator portion 204 can also have a transverse beam portion 306 perpendicularly connected to the rod or column portion and also coupled to the first jaw portion 202, the second jaw portion 208, or a combination thereof.

Where the second jaw portion 208 is movable, it would similarly have a second arm portion and a second arm hinge portion integral with the second jaw portion 208 and the actuator portion 204 respectively.

One end of the first arm portion 302 extends outwardly from the centerline line 214 to the outer periphery of the second jaw portion 208 and the other end of the first arm portion 302 is integral with the first jaw portion 202 at the first flexible hinge 216. This maximizes the length of the first arm portion 302 to maximize the mechanical advantage for moving the first jaw portion 202. It is understood that the maximum length of the first arm portion 302 is limited by a width 310, which is measured edge to edge along the second transverse dimension 212 of the connector portion 206, of the unitary jaw structure 110. In the event the unitary jaw structure 110 has a circular cross-section, the width 310 would be a diameter 310 of the circular cross-section. As an example, the ratio of the length of the first arm portion 302 from the first flexible hinge 216 to the first arm hinge portion 304 compared to the length of the first jaw portion 202 can be approximately twice as large as the ratio for a design in which a jaw is pivoted on a hinge pin intersecting the center line 214 of a jaw assembly.

It has been discovered that the arrangement of the first arm portion 302, the first flexible hinge 216, and the first arm hinge portion 304 when compared to the length of the first jaw portion 202 provides higher mechanical advantage and lower effective compliance of the actuating members 114 of FIG. 1 viewed from the jaw, thus reducing hysteresis in the motion of the jaw. This hysteresis reduction combines with the improvement gained by the low equivalent friction of the flexible hinges compared with a conventional pin joint hinge.

The first arm hinge portion 304 is a flexure or a flexible member that couples two other relatively rigid members. The first arm hinge portion 304 can be similar to the first flexible hinge 216, the second flexible hinge 218, or a combination thereof. For example, the dimensions, such as the length, and the thickness, can be optimized similar to the first and second flexible hinges. Also, for example, the first arm hinge portion 304 can be a compact flexure hinge. The first arm hinge portion 304 can couple the transverse beam portion 306 of the actuator portion 204 and the first arm portion 302.

The elimination of pivot pin friction, by the flexible jaw mechanism 308, can reduce hysteresis and increase the maximum applicable force at the first jaw portion 202 and the second jaw portion 208. The overall size of the unitary jaw structure 110 can decrease with less reduction of grip force. The resulting decrease in size can provide improved accessibility into smaller and tighter spaces through smaller incisions, which can reduce patient trauma due to the less invasive procedure.

The medical instrument 100 can have the connector portion 206 of the unitary jaw structure 110 coupled to the tube 112 of FIG. 1. The connector portion 206 and the tube 112 can be connected directly or coupled indirectly through the wrist mechanism 124 of FIG. 1. The medical instrument 100 can move the wrist mechanism 124, the tube 112, or a combination thereof to position and orient the connector portion 206 and thereby the unitary jaw structure 110. The tube 112 can hold the connector portion 206 stationary relative to the motion of the actuator portion 204.

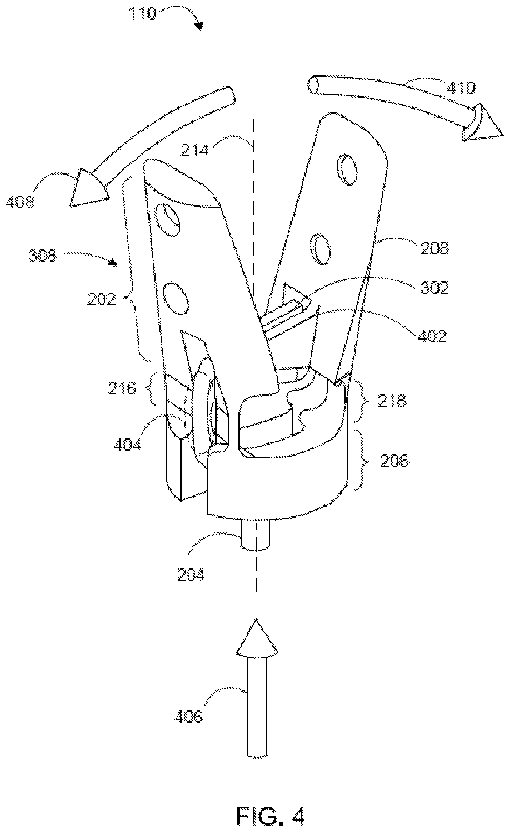

Referring now to FIG. 4, therein is shown an exemplary view of the unitary jaw structure 110 opening. The second jaw portion 208 is integral with a second arm portion 402 that is integral with a second arm hinge portion 404 that is integral with the actuator portion 204. The shape of the second arm portion 402 can be similar to the first arm portion 302. The shape of the second arm hinge portion 404 can also be similar to the first arm hinge portion 304 of FIG. 3.

The second arm portion 402 can engage the actuator portion 204 at an opposite location from where the first arm portion 302 engages the actuator portion 204.

The actuator portion 204 can be moved upward in a direction 406 to cause the first jaw portion 202 to have an outward movement 408 and the second jaw portion 208 to have an outward movement 410 for an opening of the flexible jaw mechanism 308 of the unitary jaw structure 110. The first jaw portion 202 moves in an arc around the first flexible hinge 216 and the second jaw portion 208 moves in an arc around the second flexible hinge 218. For this movement, the actuator portion 204 would be connected to or part of a rod that is one of the actuating members 114 of FIG. 1, which may be a rod or other component capable of exerting a pushing force, such as a Bowden cable.

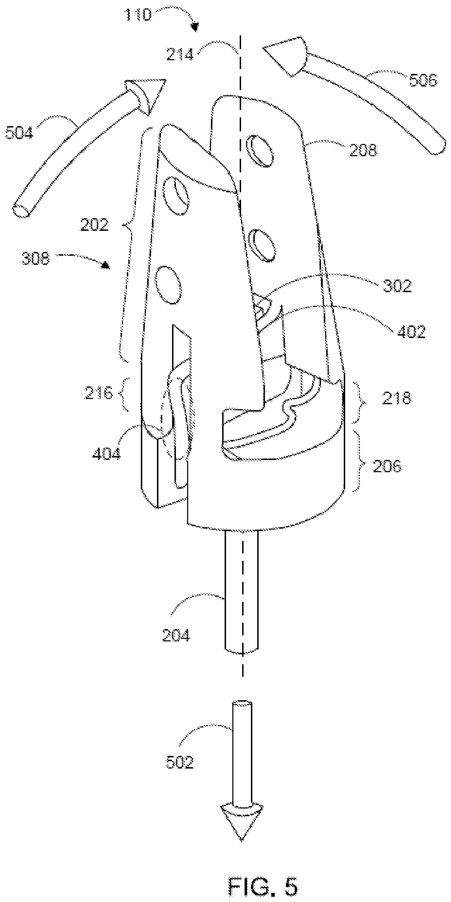

Referring now to FIG. 5, therein is shown an exemplary view of the unitary jaw structure 110 closing. The actuator portion 204 can be moved downward in a direction 502 to cause the first jaw portion 202 to have an inward movement 504 and the second jaw portion 208 to have an inward movement 506 for a closing of the flexible jaw mechanism 308 of the unitary jaw structure 110. The first jaw portion 202 moves in an arc around the first flexible hinge 216 and the second jaw portion 208 moves in an arc around the second flexible hinge 218. For this movement, the actuator portion 204 would be connected to or part of a rod that is one of the actuating members 114 of FIG. 1. Since there is only a pulling motion involved, the actuator portion 204 could be connected to a wire or cable that is one of the actuating members 114.

The resilient coupling of the first jaw portion 202 and the second jaw portion 208 by the first arm hinge portion 304 of FIG. 3 and the second arm hinge portion 404 of FIGs. 4 and 5, respectively, to the actuator portion 204 and the resilient coupling of the first jaw portion 202 and the second jaw portion 208 by the first flexible hinges 216 and the second flexible hinges 218 to the connector portion 206 can cause the inward movement 504 and the inward movement 506 if the flexible jaw mechanism 308 is open as in FIG. 4 and no force is applied to the actuator portion 204.

The mechanical spring bias, a mechanical property of the first arm hinge portion 304, to straighten at the first arm hinge portion 304 and the second arm hinge portion 404, for example, applies a bias force that causes the first jaw portion 202 and the second jaw portion 208 to close with the inward movement 504 and the inward movement 506 toward the configuration of FIG. 2.

The movement of the actuator portion 204 up and down with vertical reciprocating motion causes rotating reciprocating movement of the first jaw portion 202 and of the second jaw portion 208 about the first flexible hinges 216 and the second flexible hinges 218, respectively.

In summary, the first jaw portion 202 and the second jaw portion 208 of the flexible jaw mechanism 308 are mounted respectively on the first flexible hinge 216 and the second flexible hinge 218 which are offset to the same side of the center line 214 as each respective jaw portion rather than being pivoted on a shaft or pin intersecting the center line 214 as is done throughout the surgical instrument industry today.

In addition, the first flexible hinges 216 and the second flexible hinges 218 for each respective of the first jaw portion 202 and the second jaw portion 208 are located as far as practical off the center line 214 while the jaw portion actuating force is applied to the distal end of the first arm portion 302 and the second arm portion 402, integral with the respective of the first jaw portion 202 and the second jaw portion 208, and extending as far as practical to the opposite side of the center line 214. Additionally, the first arm hinge portion 304 and the second arm hinge portion 404 connect the first arm portion 302 and the second arm portion 402 to a common transverse beam of the actuator portion 204, which is actuated by one of the actuating members 114 fastened to the actuator portion 204 at the beam midpoint on or near the center line 214. Thus, the actuator portion 204 actuates both of the first jaw portion 202 and the second jaw portion 208 equally toward or away from each other.

The first flexible hinges 216 and the second flexible hinges 218 largely eliminate the jaw pivot pin friction torques. The effective axial stiffness for jaw motions of the actuating members 114 connected to the actuator portion 204 is greatly increased by the longer lever arm. The combined effect is to greatly reduce hysteresis manifested as lost or unpredictable motion in the unitary jaw structure 110.

It has been discovered that the first flexible hinges 216 and the second flexible hinges 218 positioned at the outer periphery of the connector portion 206 provide for longer lever arms, which reduce hysteresis that otherwise would have been manifested as lost or unpredictable motion in the unitary jaw structure 110. The reduction of friction by the flexure hinges, such as the first flexible hinges 216 and the second flexible hinges 218, enables jaw grip force sensing at a location proximal to the unitary jaw structure 110 and the tube 112 of FIG. 1. As an example, jaw force in a low friction flexure based jaw mechanism may be sensed where the actuator system 118 applies forces to the actuating member 114 which is connected to the actuator portion 204 rather than on the first jaw portion 202 and the second jaw portion 208, which has practical difficulties. In another aspect, in a low friction jaw mechanism, jaw force sensing may be based on for example, electrical current applied to a motor or pressure applied to a hydraulic or pneumatic actuator.

It has further been discovered that the first arm hinge portion 304 and the second arm hinge portion 404 also reduce hysteresis that otherwise would have been manifested as lost or unpredictable motion in the unitary jaw structure 110, which allows for the jaw force in a low friction flexure based on jaw mechanism to be sensed at the actuator portion 204 and the actuator system 118. The method of sensing at the actuator portion 204 can be similar to the methods described above for the first flexible hinges 216 and the second flexible hinges 218.

It has further yet been discovered that the first arm hinge portion 304 and the second arm hinge portion 404, which operate in tension as well as flexing to close the first jaw portion 202 and the second jaw portion 208 can be designed to limit the maximum applicable jaw force without extra sensing mechanisms. For example, the cross section area of the first arm hinge portion 304 and the second arm hinge portion 404, can be chosen based on a material with a non-linear stress-strain relation such as Nitinol such that the member will transfer only up to a threshold amount of force and deform by lengthening without increase in force when the applied force reaches the threshold maximum based on the stress-strain plateau of that alloy.

The compact flexure hinges 216, of FIG. 2, are designed to provide substantially equal shear and S-bending displacement or shear dominated displacement when a transverse force is applied to the compact flexure, as occurs in reaction to forces applied by the jaws 202 and 208 or the task adaptors 222 to patient tissue or suture needles or the like. A characteristic of the compact flexure hinges 216, when subjected to a transverse force, is that the deflection due to an S-bend component is not more than two times the shear displacement caused by the transverse force.

Embodiments of the present invention have been found to reduce the number of parts in the flexible jaw mechanism 308 from six pieces in some larger discrete component jaw mechanisms or eleven pieces in some smaller discrete component jaw mechanisms to a single integrated part that can be fabricated in a few steps of machining, such as electrical discharge machining or other practical subtractive manufacturing processes.

It has been found that the flexible jaw mechanism 308 can also be fabricated as a single piece by additive processes such as injection molding from plastic or fiber reinforced plastic composite, metal injection molding followed by sintering or by planar photo-lithographic metal plating thus reducing the part cost and the assembly time. The jaw portion flexible hinge may be made of an alloy permitting high strains such as a hardened stainless steel, titanium or aluminum alloy or from Nitinol or other more advanced shape memory alloy with even higher permissible strains or from metallic glass materials.

The working surface of each jaw portion, depending on the application, may be the parent jaw material or the task adaptor 222 of FIG. 2 made of a higher hardness alloy, tungsten carbide, toughened ceramic or finally, a suitable adhered coating such as metal plating, bonded hard grit or other deposit.

UNITARY WRIST MECHANISM

When the instrument diameter is reduced, the diameter of a hinge rotation pulley or hinge lever arm length also decreases and the required cable force increases further. The higher frictional forces and lower mechanical advantage increase cable axial deflection or stretch so that greater movement than desired of the proximal end of the actuating cable is required for a predetermined amount of distal hinge rotation, which means the effective drive train compliance is increased.

Since the hysteresis, usefully thought of as lost motion or wasted energy, of any mechanism varies with the product of the mechanism friction multiplied by its drive train compliance, the combined effect of these friction and compliance increases is a large increase in wrist motion hysteresis as the cross-sectional diameter of an instrument, such as a gripper, decreases for a given type of hinge mechanism. This is particularly detrimental when there is rubbing friction with higher starting friction that results in uneven or unpredictable motion effects sometimes called stiction.

Referring again to FIG. 1, therein is shown a medical instrument 100 with a unitary wrist structure 124. The unitary wrist structure 124 is a continuous member that bends or provides multi-axis movement to change the relative position and orientation of a member attached thereon. The term "unitary" means a structure made of a single unit of material. The unitary wrist structure 124 is a flexible member that can bend without discrete pivoting pin joints. The unitary wrist structure 124 is also supported by another member attached thereon.

For example, the unitary wrist structure 124 can be connected to a stationary member on one side and a moveable member on the opposite side. The stationary member can hold the unitary wrist structure124 in position and the unitary wrist structure 124 can be manipulated to position and orient the moveable member.

For a more specific example, the unitary wrist structure 124 can have a mechanical arm attached to one side and a camera, a gripper, or other end effectors on the other side. The mechanical arm can position the unitary wrist structure 124 with the end effector in place. The unitary wrist structure 124 can be manipulated to present different angles, orientations, and views for the camera from the given location.

The unitary wrist structure 124 can be carved or shaped out of a single unit of material, such as plastic or metal alloy. For examples, the unitary wrist structure 124 can be formed by cutting and carving polypropylene plastic or metal alloy or can be formed by using wire electrical discharge machining (EDM) process and post treatment to remove surface layer re-melt, such as when used to shape Nitinol alloy.

The unitary wrist structure 124 can also be molded into shape. For example, the unitary wrist structure 124 can be molded plastic or cast or plated metal. The unitary wrist structure 124 also can be formed as a single injection molding of polypropylene or by metal injection molding (MIM) into a die or mold that has a continuous cavity.

The medical instrument 100 has a proximal end 102 and a distal end 104. The medical instrument 100 can include the unitary wrist structure 124 near the distal end 104, a tube 112 with actuating members 114, and an actuator system 118 at the proximal end 102. The medical instrument 100 can include a jaw mechanism 110 at the distal end.

The jaw mechanism 110 can be at the distal end 104 of the medical instrument 100. The unitary wrist structure 124 can be connected to the jaw mechanism 110. The unitary wrist structure 124 can have the tube 112 attached on the other side. The unitary wrist structure 124 can also be coupled to the actuator system 118 through the actuating members 114. The jaw mechanism 110 can be analogous to a human hand, and the unitary wrist structure 124 can be analogous to a human wrist.

The jaw mechanism 110 can be a mechanical assembly, such as a gripper or a cutter, or a flexibly integral structure manufactured from a single material as a single unit. The jaw mechanism 110 and the unitary wrist structure 124 can together be continuous and integral. The jaw mechanism 110 and the unitary wrist structure 124 can both be manufactured from a single material as a single unit.

The unitary wrist structure 124 is shown having a cylindrical configuration. The cylindrical configuration eliminates sharp edges and allows the unitary wrist structure 124 and the jaw mechanism 110 to project into tight spaces and move between obstacles such as organs or blood vessels.

It has been discovered that the unitary wrist structure 124 provides for improved manufacturing operations with the advantage of eliminating assembly operations because of its one piece structure. Because of reduced cost, it is feasible to make the unitary wrist structure 124 for single use applications. A single use tool avoids the need for the handling and processing associated with cleaning and resterilization after use, as well as the need for certain instrument design requirements for resterilization, such as the ability to withstand autoclaving.

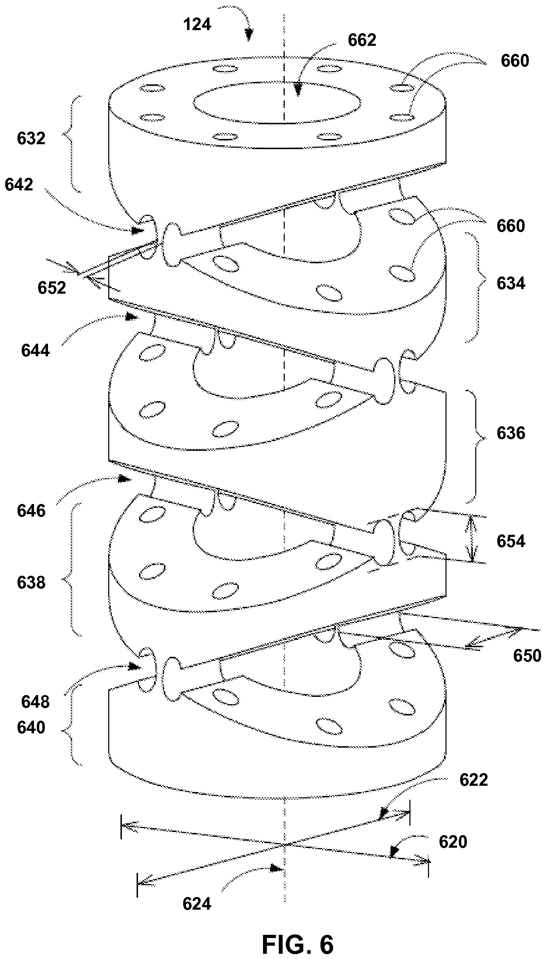

Referring now to FIG. 6, therein is shown an enlarged detailed isometric view of the unitary wrist structure 124. The unitary wrist structure 124 has a first transverse dimension 620 and a second transverse dimension 622 along a plane orthogonal to a center line 624.

The first transverse dimension 620 and the second transverse dimension 622 are shown to be the same but do not need to be and may be adjusted based on the geometry of the unitary wrist structure 124. In the case in which they are equal, the unitary wrist structure 124 may be circular in cross section as illustrated in FIG. 6. As an example, the first transverse dimension 620 and the second transverse dimension 622 are shown to be along directions perpendicular to each other but are not necessarily required to be perpendicular.

The unitary wrist structure 124 includes a first connector portion 632, a second connector portion 634, and a third connector portion 636, a fourth connector portion 638, and a fifth connector portion 640. Between the first connector portion 632 and the second connector portion 634 can be a first pair of flexible hinges 642. Between the second connector portion 634 and the third connector portion 636 can be a second pair of flexible hinges 644. Between the third connector portion 636 and the fourth connector portion 638 can be a third pair of flexible hinges 646. Between the fourth connector portion 638 and the fifth connector portion 640 can be a fourth pair of flexible hinges 648. The fifth connector portion 640 can be connectible to the tube 1 12 of FIG. 1.

Two of the pairs of flexible hinges 642-648 can be placed at right angles to two other of the pairs of flexible hinges 642-648 to allow the first connector portion 632 to be bent in all directions or omni-directionally from the fifth connector portion 640. Further, the pairs of hinges can be arranged so that the bending axes of the outermost hinges, the first pair of flexible hinges 642 and the fourth pair of flexible hinges 648, are parallel to one another. The bending axes of the innermost hinges, the second pair of flexible hinges 644 and the third pair of flexible hinges 646 can be parallel to one another. The unitary wrist structure 124 configured in this way is said to be in an ABBA configuration.

The unitary wrist structure 124 is shown having a Cartesian "yaw-pitch-pitch-yaw" or an ABBA configuration. The configuration is based on the combination of the orientation of the bending axes for the hinges as described above. The terms "yaw" and "pitch" are arbitrary terms, with the "yaw" and "pitch" describing movements in orthogonal directions. The "constant velocity" advantages for the ABBA configuration are described in more detail in U.S. Patent No. 6,817,974 (filed Jun. 28, 2002).

The pairs of flexible hinges 642-648 can have a flexure width 650 and a flexure thickness 652. The flexure width 650 is a measure of the width of the pairs of flexible hinges 642-648 along the bending axes of each pair of flexible hinges.

For example, as shown in FIG. 6, the bending axes for the first pair of flexible hinges 642 and the fourth pair of flexible hinges 648 are both parallel to the second transverse dimension 622. The flexure width 650 of the first pair of flexible hinges 642 and the fourth pair of flexible hinges 648 can be measured along lines parallel to the second transverse dimension 622. Similarly, the flexure width 650 of the second pair of flexible hinges 644 and the third pair of flexible hinges 646 can be measured along lines parallel to the first transverse dimension 620.

The flexure thickness 652 is a measure of the thickness of the pairs of flexible hinges 642-648. The flexure thickness 652 can be the measure at the thinnest point of the pairs of flexible hinges 642-648. For example, if the pairs of flexible hinges 642-648 have two elliptical concave surfaces opposing each other as shown in FIG. 6, the flexure thickness 652 can be the measure between the vertexes, or the midpoint of each of the concave surfaces.

The flexure thickness 652 can also be measured along a line perpendicular to both the bending axis of each of the pairs of flexible hinges 642-648 and the center line 624. For example, as shown in FIG. 6, the bending axes for the first pair of flexible hinges 642 and the fourth pair of flexible hinges 648 are both parallel to the second transverse dimension 622. Thus, the flexure thickness 652 of the first pair of flexible hinges 642 and the fourth pair of flexible hinges 648 can be measured along lines parallel to the first transverse dimension 620. Similarly, the flexure thickness 652 of the second pair of flexible hinges 644 and the third pair of flexible hinges 646 can be measured along lines parallel to the second transverse dimension 622.

The pairs of flexible hinges 642-648 can also have a flexure length 654. The flexure length 654 is a measure of the length or the height of the pairs of flexible hinges 642-648 along a direction parallel to the center line 624. The flexure length 654 can be measured when the pairs of flexible hinges 642-648 are at a neutral position as shown in FIG. 6. The flexure length 654 can be the distance between the points where the pairs of flexible hinges 642-648 are integral with the adjoining connector portions 632-640.

The pairs of flexible hinges as illustrated in FIG. 6 are typical of flexible hinges and compact flexures. A flexure or flexible hinge is defined as a flexible member that couples two other relatively rigid members. The pairs of flexible hinges 642-648 can be flexible members coupling the connector portions 632-640 that are relatively rigid members.

The pairs of flexible hinges 642-648 can have the flexure thickness 652 that is sufficiently less than the flexure length 654 for providing a bending compliance between the coupled rigid members allowing one rigid member to rotate with respect to the other about an axis along the flexure width 650. The bending axis is located at the midpoint of the flexure length 654 and the flexure thickness 652 when the flexure is straight.

The flexure length 654 and the flexure thickness 652 can have a ratio of length to thickness such that the bending strain and the resulting stress in the flexure are within limits based on its material properties, such as yield strength or fatigue strength, its angular range of motion, and the required flexing motion cycles. The pairs of flexible hinges 642-648 may rigidly transmit forces along at least the flexure width 650 and the flexure length 654 between the two coupled members.

It has been discovered that the pairs of flexible hinges 642-648, when moving, have only low internal hysteresis losses in the material, also called equivalent friction, which are significantly lower than the corresponding actual friction losses in a similarly loaded pin jointed hinge. Hysteresis loss is the loss of motion or energy in mechanisms and structures due to the material properties and mechanical configuration thereof.

The pairs of flexible hinges 642-648 can also be compact flexures. A compact flexure is a flexure made from a plastic material such as injection molded polypropylene or ultra-high molecular weight polyethylene (UHMW-PE) whose material properties permit a short flexure length while at the same time permitting a high number of flexing motion cycles. For example, the flexure length 654 can be less than twice the flexure thickness 652.

The flex life is defined to be the number of times a flexible hinge 642-648 can be reliably flexed or bent without breaking. The flex life of the pairs of compact flexures 642-648 can be enhanced by providing that the melted plastic flows through the hinge from one coupled rigid member toward the other as the mold fills and by flexing the hinge fully in both directions immediately upon removal from the mold. Because of the reduction in the flexure length 654, in relation to the flexure thickness 652, the pairs of compact flexures 642-648 advantageously provide relatively greater stiffness than conventional flexures with respect to forces between the coupled members in the direction of the flexure thickness 652 as may occur when a transverse load along direction of the flexure thickness 652 or a torsional load about the center line 624 is applied between adjacent pairs of coupled members 632 through 640. Also advantageously, the molded plastic compact flexure may reduce cost by eliminating multiple separate components and associated assembly labor as well as by using a low cost material.

In addition to plastic or metal injection molding or cutting of plastic or metal by machining methods, including metal cutting by wire electrical discharge machining (EDM), the pairs of flexible hinges 642-648 may be configured with sufficient length and uniform thickness to permit exploitation of a planar photo-lithographic plated metal alloy fabrication process by orienting the flexible hinges in the plane of the plated layers to enable unitary structures of 1 mm diameter or smaller. Thus, the invention enables manufacture of functional medical flexible hinge wrist instruments of unprecedented smaller size and further enables use in surgery on correspondingly smaller anatomy such as re-anastomosis of small blood vessels and nerves or manipulation and repair of structures inside the eye.

The flexible hinge portions can be resilient. Therefore, the release of the actuating members 114 of FIG. 1 where there is no pull or push force on the connector portions 632-640 can cause the connector portions 632-640 to move to a position where the pairs of flexible hinges 642-648 are at a defined neutral position. For example, the pairs of flexible hinges 642-648 can be biased to return to generally a straight and extended formation, substantially parallel to one another or to a predetermined bent shape.

The first connector portion 632, the second connector portion 634, the third connector portion 636, the fourth connector portion 638, and the fifth connector portion 640 all have a plurality of actuator holes 660 that are in the periphery of each connector portion and run parallel to the center line 624.

The actuator holes 660 are shown around the periphery of the unitary wrist structure 124. The actuator holes 660 are aligned through the unitary wrist structure 124. In one aspect, the actuator holes 660 go through each connector portion. In another aspect, some of the actuator holes 660 may pass through only some of the connector portions. In one example, some of the actuator holes 660 may pass through only the connector portions 640, 638 and 636 when they will only be used for actuating members to move connector portions 638 and 636 with respect to the fifth connector portion 640.

The actuating members 114 of FIG. 1 that are used to control the unitary wrist structure 124, in the form of control wires or miniature wound or braided cables or ropes, are threaded through the unitary wrist structure 124 through the actuator holes 660 to each of the connector portions. The tension on the actuating members 114 can cause the unitary wrist structure 124 to bend at the pairs of flexible hinges. Depending on the placement of the wrist control wires in the actuator holes 660 and the relative displacement of the actuating members 114, the unitary wrist structure 124 can bend with two to four degrees of freedom although two is a preferred case.

A central channel 662 extends along the center line 624 of the unitary wrist structure 124 for passage of the one of the actuating members 114 for activating the jaw mechanism 110 connected to the unitary wrist structure 124.

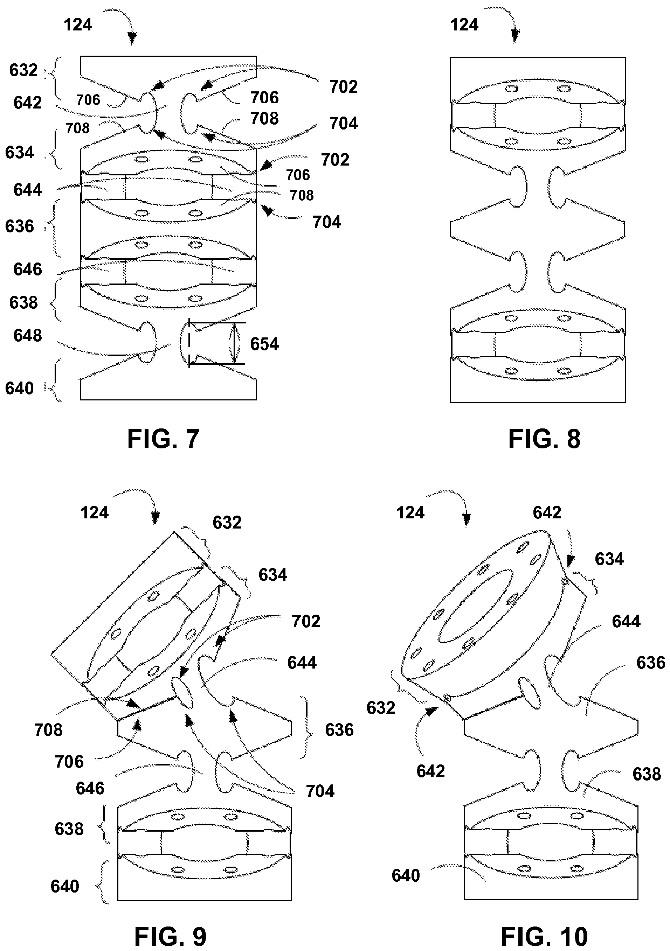

Referring now to FIG. 7, therein is shown an enlarged front view of the unitary wrist structure 124. The connector portions 632-638 can have lower relief clearances 702 on the lower portions thereof. The connector portions 634-640 can have upper relief clearances 704 on the upper portions thereof.

The pairs of flexible hinges 642-648 can be between adjacent connector portions. For example, the first pair of flexible hinges 642 can be integral with the lower relief clearances 702 on the lower portion of the first connector portion 632. The first pair of flexible hinges 642 can also be integral with the upper relief clearances 704 on the upper portion of the second connector portion 634. The pairs of flexible hinges 644-648 can similarly provide integral connection between connector portions 634-640.

For illustrative purposes, the pairs of flexible hinges 642-648 are shown connecting to the lower relief clearances 302 at the upper ends of each hinge and the upper relief clearances 704 at the lower ends of each hinge. However, it is understood that the unitary wrist structure 124 can have a different orientation, thereby changing the relative positions of the lower relief clearances 702 and the upper relief clearances 704 and the hinges.

The lower relief clearances 702 and the upper relief clearances 704 are the indentations in connector portions 632-640 at the junction between the each of the connector portions 632-640 and each of the pairs of flexible hinges 642-648. For example, the lower relief clearances 702 can be radii by the first pair of flexible hinges 642 at the junction with the first connector portion 632.

Continuing with the example, the lower relief clearances 702 and the upper relief clearances 704 can be a portion of an ellipse around the vertex point on the major axis of the ellipse while the midpoint of the flexure is at the co-vertex on the minor axis of the ellipse. As a more specific example, the lower relief clearances 702 and the upper relief clearances 704, together with a surface on each of the hinges can form a portion of a cylinder having an elliptical cross-section. The lower relief clearances 702 and the upper relief clearances 704 allow a reduction in the vertical height of the unitary wrist structure 124. The lower relief clearances 702 and the upper relief clearances 704 can be integral with the pairs of flexible hinges 642-648 at the ends of the major axes of the elliptical cross-section. The point for dividing the portions of the hinges and the clearances can be the vertex points of the elliptical cross-section. Further, the flexure length 654 of each hinge can be the length of the major axes of the elliptical cross-section, measured from one vertex to another along a line parallel to the center line 624 of FIG. 6.

The connector portions 632-638 can have lower stop surfaces 706 on the lower portions thereof. The connector portions 634-640 can have upper stop surfaces 708 on the upper portions thereof. The lower relief clearances 702 can be adjacent to the lower stop surfaces 706 and the upper relief clearances 704 can be adjacent to the upper stop surfaces 708. The lower stop surfaces 706 are planar surfaces on the lower portions of the connector portions 632-638 for restricting the movement of the connector portions 632-638 and the bend of the pairs of flexible hinges 642-648. The upper stop surfaces 708 are planar surfaces on the upper portions of the connector portions 632-638 for restricting the movement of the connector portions 634-640 and the bend of the pairs of flexible hinges 642-648.

For illustrative purposes, the lower stop surfaces 706 are shown as being the under surfaces of the connector portions 632-638 and the upper stop surfaces 708 as being on the upper surfaces of the connector portions 634-640. However, it is understood that the unitary wrist structure 124 can be oriented in different positions, thereby changing the relative positions of the lower stop surfaces 706 and the upper stop surfaces 708.

The first connector portion 632 can have a pair of the lower stop surfaces 706 only. The fifth connector portion 640 can have a pair of the upper stop surfaces 708 only. The connector portions 634-638 can have both a pair of the lower stop surfaces 706 and a pair of the upper stop surfaces 708.

For the second connector portion 634, the third connector portion 636, and the fourth connector portion 638, each can have the upper stop surfaces 708 and the lower stop surfaces 706 positioned on top of each other and offset by 90 degrees about the center line 624, or offset by a different angle. For example, the second connector portion 634 is shown having the upper stop surfaces 708 on the left and right side of the second connector portion 634. The lower stop surfaces 706 are shown arranged with 90 degree offset and are positioned on the front and the back side.

Continuing with the example, the third connector portion 636 is shown having both the upper stop surfaces 708 and the lower stop surfaces 706 arranged directly on top of each other without angular offset about the center line 624. Both the upper stop surfaces 708 are shown positioned on the front and the back side (not shown) of the unitary wrist structure 124.

The lower stop surfaces 706 of one connector portion can be directly over, above, facing, or mirroring the upper stop surfaces 708 of the connector portion below. For example, the lower stop surface 706 of the first connector portion 632 can be directly over and above and mirroring the upper stop surfaces 708 of the second connector portion 634. Also, for example, the second connector portion 634 can have the lower stop surface 706 directly over and mirroring the upper stop surface 708 of the third connector portion 636.

The lower stop surfaces 706 and the upper stop surfaces 708 can be angled away from a horizontal plane for restricting the movement of the connector portions 632-638 and the bend of the pairs of flexible hinges 642-648. The lower stop surfaces 706 can connect to the lower relief clearances 702 of the pairs of flexible hinges 642-648 and extend upwards and away from the pairs of flexible hinges 642-648 at a predetermined angle. The upper stop surfaces 708 can connect to the upper relief clearances 704 of the pairs of flexible hinges 642-648 and extend downwards and away from the pairs of flexible hinges 642-648 at a predetermined angle.

Referring now to FIG. 8, therein is shown an enlarged side view of the unitary wrist structure 124. The unitary wrist structure 124 can have identical shapes between front and back and between left and right.

For purposes of discussion, the unitary wrist structure 124 as shown in FIG. 7 will be discussed as facing left in FIGs. 8-10. In other words, FIGs. 8-10 will be discussed as having the right side of the unitary wrist structure 124 in FIG. 7 illustrated. However, it is understood that FIG. 8 can be the left or the right side of the unitary wrist structure 124.

Referring now to FIG. 9, therein is shown an enlarged side view of the unitary wrist structure 124 partially flexed forward. The unitary wrist structure 124 is shown having the second pair of flexible hinges 644 fully flexed forward.

The first connector portion 632 and the second connector portion 634 are shown leaning forward. The connector portions 636-640 have the same neutral orientation as in FIG. 8.

The unitary wrist structure 124 is shown having the lower stop surface 706 on the front side of the second connector portion 634 abutting the upper stop surface 708 on the front side of the third connector portion 636. The two abutting surfaces can overlap each other and cover the entire surfaces. The two abutting surfaces stop the second pair of flexible hinges 644 from bending forward further. The unitary wrist structure 124 can fully flex forward by similarly bending the third pair of flexible hinges 646 forward in a similar manner. The medical instrument 100 of FIG. 1 can use the actuating members 114 of FIG. 1 to flex the unitary wrist structure 124.

It has been discovered that the slope of the lower stop surfaces 706 and the upper stop surfaces 708 can be used to limit the amount of bend in the unitary wrist structure 124 without further device or limiting mechanism. The slope of the lower stop surfaces 706 and the upper stop surfaces 708 can thusly eliminate external limiters, such as gear mechanism or limitation feature in software, and internal limiters, such as bumps or stoppers, and simplify manufacturing complexity and reduce manufacturing cost.

The lower relief clearance 702 and the upper relief clearance 704 can form a cylinder having an elliptical cross-section as shown on the front side of the second pair of flexible hinges 644. For example, the lower relief clearance 702 and the upper relief clearance 704 can open up or enlarge the arc portion of the lower relief clearances 702 and the upper relief clearance 704 on the backside of the second pair of flexible hinges 644.

The material in the front side of the second pair of flexible hinges 644 compresses when the second pair of flexible hinges 644 bends forward. The material in the backside of the second pair of flexible hinges 644 stretches when the second pair of flexible hinges 644 bends forward.

The slopes of the abutting pair of the upper stop surface 708 and the lower stop surface 706 can limit the bend of the pairs of flexible hinges. For example, the magnitude of the angle away from a horizontal plane for the upper stop surface 708 can be added with that of the lower stop surface 706. The combined angles can be the maximum amount of bend for a given pair of flexible hinges.

Referring now to FIG. 10, therein is shown an enlarged side view of the unitary wrist structure 124 partially flexed forward and partially flexed to the right. The unitary wrist structure 124 is shown flexed forward as described above. The unitary wrist structure 124 is shown also similarly flexed partially to the right, as viewed from the front in the reference frame of FIG. 7.

The second connector portion 634 has the same position as shown in FIG. 9. The connector portions 636-640 have the same neutral position as shown in FIG. 8. The first connector portion 632 is shown rotated to its left.

The lower stop surface 706 of FIG. 7 on the left side of the first connector portion 632 can abut the upper stop surface 708 of FIG. 7 on the left side of the second connector portion 634. The first pair of flexible hinges 642 can flex and bend to the left, similar to the second pair of flexible hinges 644 as described above.

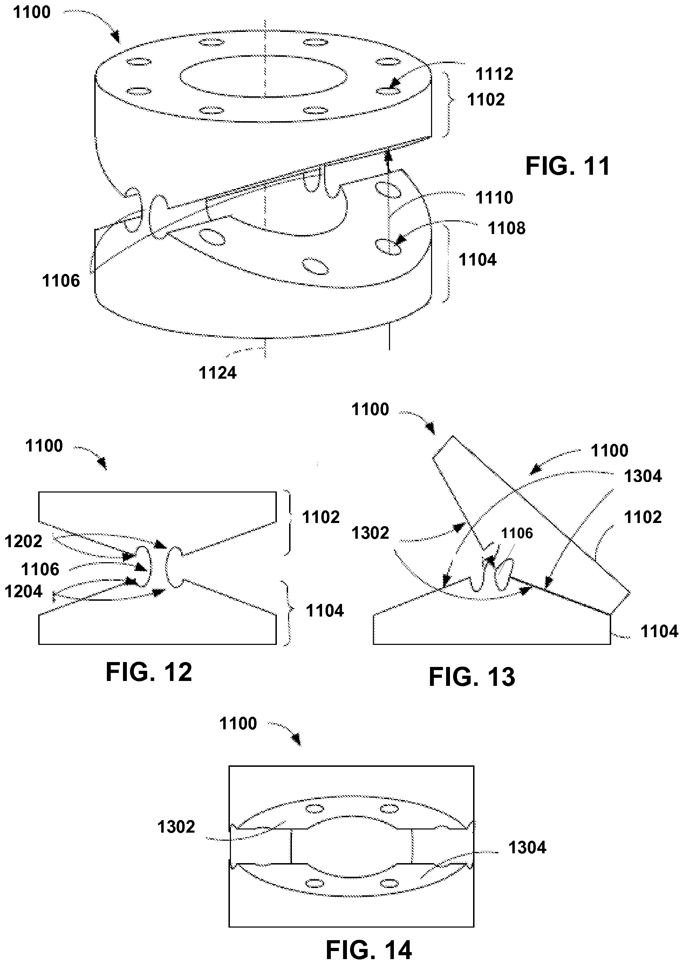

Referring now to FIG. 11, therein is shown an enlarged detailed isometric view of a unitary joint structure 1100. A first connector portion 1102 is similar to the first connector portion 632 of FIG. 6 and a second connector portion 1104 is similar to the fifth connector portion 640 of FIG. 6. The first connector portion 1102 could be connected to or integral with the jaw mechanism 110 of FIG. 1 and the second connector portion 1104 could be connected to the tube 112 of FIG. 1.

Between the first connector portion 1102 and the second connector portion 1104 are a pair of flexible hinges 1106. The pair of flexible hinges 1106 is similar to the first pair of flexible hinges 642 of FIG. 6.

The actuating members 114 of FIG. 1 can slide through second actuator holes 1108 along a line 1110 and can be fastened in first actuator holes 1112. By pulling on the actuating members 1114 on one side or another of the pair of flexible hinges 1106, the unitary joint structure 1100 will bend in one direction or another. This principle is also applicable, to a greater extent, to the unitary wrist structure 124 of FIG. 6.

Referring now to FIG. 12, therein is shown a front view of the unitary joint structure 1100. The pair of flexible hinges 1106 connects the first connector portion 1102 and the second connector portion 1104.

The first connector portion 1 102 can have lower relief clearances 802 on the lower portion thereof. The second connector portion 1104 can have upper relief clearances 1204 on the upper portion thereof.

The pair of flexible hinges 1106 can be connected to the lower relief clearances 1202 at one end and the upper relief clearances 1204 at the opposite end. The lower relief clearances 1202 and the upper relief clearances 1204 can be similar to the lower relief clearances 702 and the upper relief clearances 704 of FIG. 7. The lower relief clearances 1202 and the upper relief clearances 1204 allow a reduction in the vertical height of the unitary joint structure 1100 by permitting the combined connector portions and flexible hinge to fit in a smaller overall height.

Referring now to FIG. 13, therein is shown a front view of the unitary joint structure 1100 in a fully flexed position. The first connector portion 1102 can have lower stop surfaces 1302 on the lower portion thereof and the second connector portion 1104 can have upper stop surfaces 1304 on the upper portion thereof. In the fully flexed position, the lower stop surfaces 1302 of the first connector portion 1102 abut the upper stop surfaces 1304 of the second connector portion 1104.

The lower stop surfaces 1302 can be similar to the lower stop surfaces 706 of FIG. 7. The upper stop surfaces 1304 can be similar to the upper stop surfaces 708 of FIG. 7. The lower stop surfaces 1302 and the upper stop surfaces 1304 are angled to limit the amount of bend of the pair of flexible hinges 1106 and of the unitary joint structure 1100.

It has been discovered that having the stop surfaces assures the pair of flexible hinges 1106 stay within design parameters to assure adequate life of the pair of flexible hinges 1106. When the stop surfaces are used in the unitary wrist structure 124 of FIG. 1, it also has been found to prevent the actuating members 114 from bending excessively at the mouths of the actuator holes 660 and causing excessive friction and wear.

The pair of flexible hinges 1106 can bend in the middle portion or stretch to accommodate the flexed position. For example, the middle portion of the pair of flexible hinges 1106 can stretch on one side and compress on the other side. The lower relief clearances 1202 of FIG. 12, the upper relief clearances 1204 of FIG. 12 and the stretched surface of the pair of flexible hinges 1106 can form a cross-section shaped like the numeral '3' or they can form an elliptical cross section. On the opposite side, the lower relief clearances 1202, the upper relief clearances 1204 and the compressed surface of the pair of flexible hinges 1106 can form a triangular or a heart-shaped cross-section. Also, for example, the pair of flexible hinges 1106 can stretch or compress evenly and retain the overall oval shape as exemplified in FIGs. 9 and 10.

Referring now to FIG. 14, therein is shown a side view of the unitary joint structure 1100. The upper stop surface 1304 is shown directly under and mirroring the lower stop surface 1302.

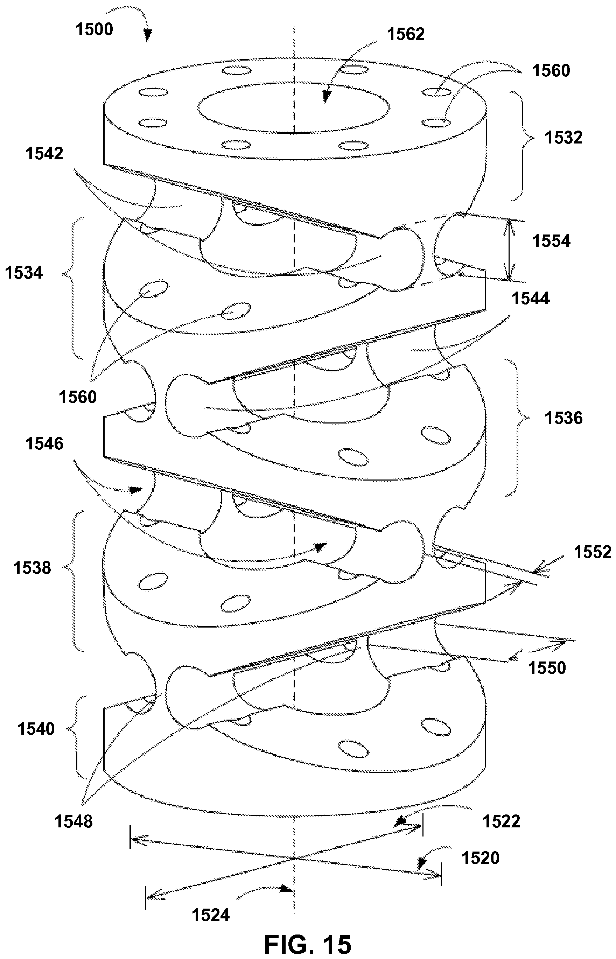

Referring now to FIG. 15, therein is shown an enlarged detailed isometric view of a unitary wrist structure 1500. The unitary wrist structure 1500 has a first transverse dimension 1520 and a second transverse dimension 1522 along a plane orthogonal to a center line 1524. The first transverse dimension 1520 and the second transverse dimension 1522 are shown to be the same but do not need to be and may be adjusted based on the geometry of the unitary wrist structure 1500. In the case in which they are equal, the unitary wrist structure 1500 may be circular in cross section as illustrated in FIG. 15.

The unitary wrist structure 1500 includes a first connector portion 1532, a second connector portion 1534, and a third connector portion 1536, a fourth connector portion 1538, and a fifth connector portion 1540. Between the first connector portion 1532 and the second connector portion 1534 is a first pair of flexible hinges 1542. Between the second connector portion 1534 and the third connector portion 1536 is a second pair of flexible hinges 1544. Between the third connector portion 1536 and the fourth connector portion 1538 is a third pair of flexible hinges 1546. Between the fourth connector portion 1538 and the fifth connector portion 1540 is a fourth pair of flexible hinges 1548. The fifth connector portion 1540 is connectible to the tube 112 of FIG. 1.

Two of the pairs of flexible hinges can be placed at right angles to two other of the pairs of flexible hinges to allow the first connector portion 1532 to be bent in all directions or omni-directionally from the fifth connector portion 1540. Further, the pairs of flexible hinges can be arranged alternating 90 degrees so that the bending axes of the first pair of flexible hinges 1542 and the third pair of flexible hinges 1546 are parallel to one another. The bending axes of the second pair of flexible hinges 1544 and the fourth pair of flexible hinges 1548 can be parallel to one another. The unitary wrist structure 1500 configured in this way is said to be in an ABAB configuration.

The unitary wrist structure 1500 is shown having a Cartesian "yaw-pitch-yaw-pitch" or the ABAB configuration. The configuration is based on the combination of the orientation of the bending axes for the hinges as described above. The terms "yaw" and "pitch" are arbitrary terms, with the "yaw" and "pitch" describing movements in orthogonal directions.

By this alternation, further nesting the structural features permits reduction of the length to diameter ratio L / D from L / D = 2 in the ABBA case to as little as L / D = 1.8 in the ABAB case. In the ABAB case, errors in rotational motion of the first connector portion 1532 relative to the fifth connector portion 1540 occur when the fifth connector portion 1540 is rotated about the center line 1524 and the first connector portion 1532 maintains a fixed pointing direction. This is called wrist roll motion. The rotational motion errors of the first connector portion 1532 may be compensated by kinematic computation.

The pairs of flexible hinges 1542-1548 can have a flexure width 1550 and a flexure thickness 1552. The flexure width 1550 is a measure of the width of the pairs of flexible hinges 1542-1548 along the bending axes of each pair of flexible hinges.

For example, as shown in FIG. 15, the bending axes for the first pair of flexible hinges 1542 and the third pair of flexible hinges 1546 are both parallel to the first transverse dimension 1520. The flexure width 1550 of the first pair of flexible hinges 1542 and the third pair of flexible hinges 1546 can be measured along lines parallel to the first transverse dimension 1520. Similarly, the flexure width 1550 of the second pair of flexible hinges 1544 and the fourth pair of flexible hinges 1548 can be measured along lines parallel to the second transverse dimension 1522.

The flexure thickness 1552 is a measure of the thickness of the pairs of flexible hinges 1542-1548. The flexure thickness 1552 can be the measure at the thinnest point of the pairs of flexible hinges 1542-1548. For example, if the pairs of flexible hinges 1542-1548 have two concave surfaces opposing each other as shown in FIG. 15, the flexure thickness 1552 can be the measure between the vertexes of each of the concave surfaces.

The flexure thickness 1552 can also be measured along a line perpendicular to both the bending axis of each of the pairs of flexible hinges 1542-1548 and the center line 1524. For example, as shown in FIG. 15, the bending axes for the first pair of flexible hinges 1542 and the third pair of flexible hinges 1546 are both parallel to the first transverse dimension 1520. Thus, the flexure thickness 1552 of the first pair of flexible hinges 1542 and the third pair of flexible hinges 1546 can be measured along lines parallel to the second transverse dimension 1522. Similarly, the flexure thickness 1552 of the second pair of flexible hinges 1544 and the fourth pair of flexible hinges 1548 can be measured along lines parallel to the first transverse dimension 1520.

The pairs of flexible hinges 1542-1548 can also have a flexure length 1554. The flexure length 1554 is a measure of the length or the height of the pairs of flexible hinges 1542-1548 along a direction parallel to the center line 1524. The flexure length 1554 can be measure when the pairs of flexible hinges 1542-1548 are at a neutral position as shown in FIG. 151. The flexure length 1554 can be the distance between the points where the pairs of flexible hinges 1542-1548 are integral with the adjoining connector portions 1532-1540.

The pairs of flexible hinges as illustrated in FIG. 15 are typical of flexible hinges and compact flexures. A flexure or flexible hinge is defined as a flexible member that couples two other relatively rigid members. The pairs of flexible hinges 1542-1548 can be flexible hinges coupling the connector portions 1532-1540 that are relatively rigid members.

The pairs of flexible hinges 1542-1548 can each have the flexure thickness 1552 that is sufficiently less than the flexure length 1554 for providing a bending compliance between the coupled rigid members allowing one rigid member to rotate with respect to the other about an axis parallel to the flexure width 1550. The bending axis can be located at the midpoint of the flexure thickness 1552 when the flexure is straight.

The flexure length 1554 and the flexure thickness 1552 can have a ratio of length to thickness such that the bending strain and the resulting stress in the flexure are within limits based on its material properties, such as yield strength or fatigue strength limit, angular range of motion, and required flexing motion cycles. The pairs of flexible hinges 1542-1548 may rigidly transmit forces along at least the flexure width 1550 between the two coupled members and preferably along the flexure thickness 1552 and the flexure length 1554.

It has been discovered that the pairs of flexible hinges 1542-1548, when moving, have only low internal hysteresis losses in the material, also called equivalent friction, which are significantly lower than the corresponding actual friction losses in a similarly loaded pin jointed hinge. Hysteresis loss is the loss of motion or energy in mechanisms and structures due to the material properties and mechanical configuration thereof.

The pairs of flexible hinges 1542-1548 can also be compact flexures. A compact flexure is a flexure made from a plastic material such as injection molded polypropylene or ultra-high molecular weight polyethylene (UHMW-PE) whose material properties permit a short flexure length while at the same time permitting a high number of flexing motion cycles. For example, the flexure length 1554 can be less than twice the flexure thickness 1552.

The flex life of the pairs of flexible hinges 1542-1548 can be enhanced by providing that the melted plastic flows through the hinge from one coupled rigid member toward the other as the mold fills and by flexing the hinge fully in both directions immediately upon removal from the mold. Because of its reduced length in relation its thickness, the pairs of flexible hinges 1542-1548 advantageously provide relatively greater stiffness than conventional flexures with respect to forces between the coupled members in the direction of its thickness and with respect to torques about the center line 1524. Also advantageously, the compact flexure may reduce cost by eliminating multiple separate components and associated assembly labor as well as by using a low cost material.

In addition to plastic or metal injection molding or cutting of plastic or metal by machining methods, including metal cutting by wire EDM, the pairs of flexible hinges 1542-1548 may be configured with greater length and uniform thickness to permit exploitation of a planar photo-lithographic plated metal alloy fabrication process by orienting the flexible hinges in the plane of the plated layers to enable unitary structures of 1 mm diameter or smaller. Thus, the invention enables manufacture of functional medical flexible hinge wrist instrument of unprecedented smaller size and further enables use in surgery on correspondingly smaller anatomy such as re-anastomosis of small blood vessels and nerves or manipulation and repair of structures inside the eye.

The first connector portion 1532, the second connector portion 1534, the third connector portion 1536, the fourth connector portion 1538, and the fifth connector portion 1540 all have a plurality of actuator holes 1560 that are in the periphery of each connector portion and run parallel to the center line 1524.

The actuator holes 1560 are shown around the periphery of the unitary wrist structure 1500. The actuator holes 1560 are aligned through the unitary wrist structure 1500. In one aspect, the actuator holes 1560 go through each connector portion. In another aspect, some of the actuator holes 1560 may pass through only some of the connector portions. In one example, some of the actuator holes 1560 may pass through only connector portions 1540, 1538 and 1536 when they will only be used for actuating members to move connector portions 1538 and 1536 with respect to the fifth connector portion 1540.

The actuating members 114 of FIG. 1 that are used to control the unitary wrist structure 1500, in the form of control wires or miniature wound or braided cables or ropes, are threaded through the unitary wrist structure 1500 through the actuator holes 1560 to each of the connector portions. The tension on the actuating members 114 can cause the unitary wrist structure 1500 to bend at the pairs of flexible hinges. Depending on the placement of the wrist control wires in the actuator holes 1560 and the relative displacement of the actuating members 114, the unitary wrist structure 1500 can bend with two to four degrees of freedom although two is a preferred case.

A central channel 1562 extends along the center line 1524 of the unitary wrist structure 1500 for passage of one of the actuating members 114 for activating the jaw mechanism 110 connected to the unitary wrist structure 1500.

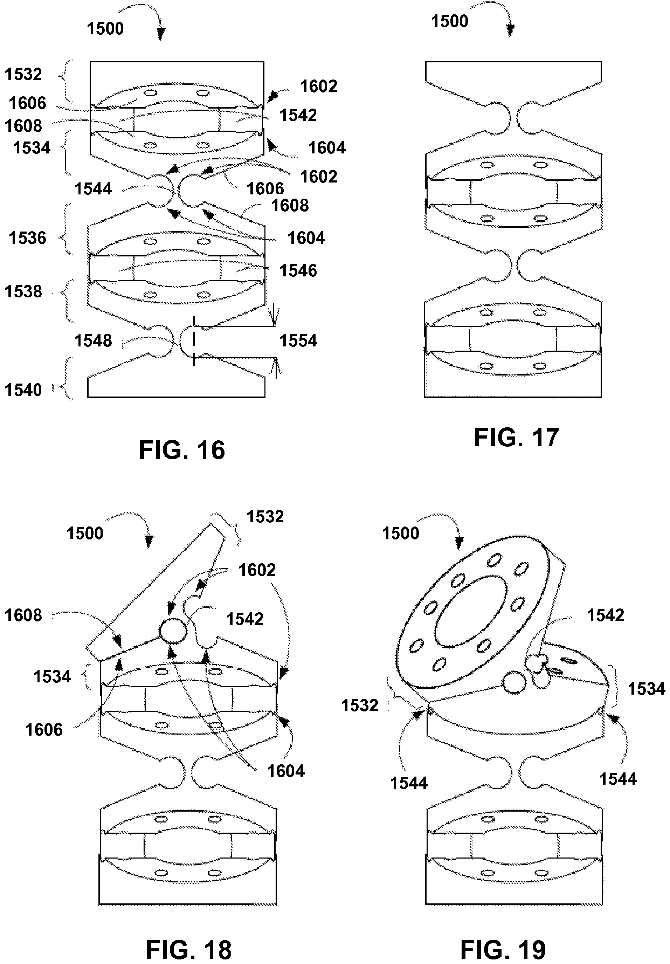

Referring now to FIG. 16, therein is shown an enlarged front view of the unitary wrist structure. The connector portions 1532-1538 can have lower relief clearances 1602 on the lower portions thereof. The connector portions 1534-1540 can have upper relief clearances 1604 on the upper portions thereof.

The pairs of flexible hinges 1542-1548 can be between adjacent connector portions. For example, the first pair of flexible hinges 1542 can be integral with the lower relief clearances 1602 on the lower portion of the first connector portion 1532. The first pair of flexible hinges 1542 can also be integral with the upper relief clearances 1604 on the upper portions of the second connector portion 1534. The pairs of flexible hinges 1544-1548 can similarly provide integral connection between connector portions 1534-1540.

For illustrative purposes, the pairs of flexible hinges 1542-1528 are shown connecting to the lower relief clearances 1602 at the upper ends of each hinge and the upper relief clearances 1604 at the lower ends of each hinge. However, it is understood that the unitary wrist structure 1500 can have a different orientation, thereby changing the relative positions of the lower relief clearances 1602 and the upper relief clearances 1604 and the hinges. The lower relief clearances 1602 and the upper relief clearances 1604 are the indentations in the connector portions 1532-1540 that are at the junction between the each of the connector portions 15132-1540 and each of the pairs of flexible hinges 1542-1548. For example, the lower relief clearances 1602 can be an arc or a portion of a circular indentation or depression in the first connector portion 1532 at the junction with the first pair of flexible hinges 1542.

Continuing with the example, the lower relief clearances 1602 and the upper relief clearances 1604 can be an arc or a portion of a circular indentation or depression integral with a surface of the opposing pairs of connector portions 1532-1540. The lower relief clearances 1602 and the upper relief clearances 1604, together with a surface of each of the hinges can form a continuous portion of a cylinder having a circular cross-section.

The lower relief clearances 1602 and the upper relief clearances 1604 can be integral with the pairs of flexible hinges 1542-1548 at the ends of the diameter parallel with the center line 1524 of FIG. 15 on the circular cross-section. The point for dividing the portions of the hinges and the clearances can be at the ends of the diameter parallel with the center line on the circular cross-section. Further, the flexure length 1554 of each hinge can be the diameter of the circular cross-section.

The lower relief clearances 1602 and the upper relief clearances 1604 can be connected to lower stop surfaces 1606 and upper stop surfaces 1608. The lower relief clearances 1602 can be connected to the lower stop surfaces 1606 and the upper relief clearances 1604 can be connected to the upper stop surfaces 1608.

The lower stop surfaces 1606 are planar surfaces on the lower portions of the connector portions 1532-1538 for restricting the movement of the connector portions 1532-1538 and the bend of the pairs of flexible hinges 1542-1548. The upper stop surfaces 1608 are planar surfaces on the upper portions of the connector portions 1534-1540 and mirroring the lower stop surfaces 1606 for abutting the lower stop surfaces 1606 to restrict the movement of the connector portions 1532-1538 and the bend of the pairs of flexible hinges 1542-1548.

For illustrative purposes, the lower stop surfaces 1606 are shown as being the lower portion of the connector portions 1532-1538 and the upper stop surfaces 1608 as being the upper portion of the connector portions 1534-1540. However, it is understood that the unitary wrist structure 1500 can be oriented differently, thereby changing the relative positions of the lower stop surfaces 1606 and the upper stop surfaces 1608, the connector portions 1532-1540, and the hinges.

The first connector portion 1532 can have a pair of the lower stop surfaces 1606 only. The fifth connector portion 1540 can have a pair of the upper stop surfaces 1608 only. The connector portions 1534-1538 can have both a pair of the lower stop surfaces 1606 and a pair of the upper stop surfaces 1608.

For the second connector portion 1534, the third connector portion 1536, and the fourth connector portion 1538 each can have the upper stop surfaces 1608 rotationally offset about the center line 1524 from the lower stop surfaces 1606 by 90 degrees or offset by a different angle. For example, the second connector portion 1534 is shown having the upper stop surfaces 1608 on the front and back (not shown) side of the second connector portion 1534. The lower stop surfaces 1606 are shown arranged with 90 degree offset and are positioned on the left side and the right side.

The lower stop surfaces 1606 of one connector portion can be directly over, above, facing, or mirroring the upper stop surfaces 1608 of the connector portion below. For example, the lower stop surface 1606 of the first connector portion 1532 can be directly over and above and also mirror the upper stop surfaces 1608 of the second connector portion 1534. Also, for example, the second connector portion 1534 can have the lower stop surface 1606 directly over and above the upper stop surface 1608 of the third connector portion 1536, with the two sets of surfaces mirroring each other.

The lower stop surfaces 1606 and the upper stop surfaces 1608 can be angled away from a horizontal plane for restricting the movement of the connector portions 1532-1538 and the bend of the pairs of flexible hinges 1542-1548. The lower stop surfaces 1606 can connect to the lower relief clearances 1602 of the pairs of flexible hinges 1542-1548 and extend upwards and away from the pairs of flexible hinges 1542-1548 at a predetermined angle.

Referring now to FIG. 17, therein is shown an enlarged side view of the unitary wrist structure. The unitary wrist structure 1500 can have identical shapes between front and back and left and right.

For purposes of discussion, the unitary wrist structure 1500 as shown in FIG. 16 will be discussed as facing left in FIGs. 17-19. In other words, FIGs. 17-19 will be discussed as having the right side of the unitary wrist structure 1500 in FIG. 16 illustrated. However, it is understood that FIG. 17 can be the left or the right side of the unitary wrist structure 1500.

Referring now to FIG. 18, therein is shown an enlarged side view of the unitary wrist structure partially flexed forward. The unitary wrist structure 1500 is shown having the first pair of flexible hinges 1542 fully flexed forward.

The first connector portion 1532 is shown rotated forward. The connector portions 1534-1538 of FIG. 15 have the same neutral orientation as in FIG. 16.

The unitary wrist structure 1500 is shown having the lower stop surface 1606 on the front side of the first connector portion 1532 abutting the upper stop surface 1608 on the front side of the second connector portion 1534. The two abutting surfaces stop the first pair of flexible hinges 1542 from bending forward further. The unitary wrist structure 1500 can fully flex forward by similarly bending the third pair of flexible hinges 1546 of FIG. 16 forward in a similar manner. The medical instrument 100 of FIG. 1 can use the actuating members 114 of FIG. 1 to flex the unitary wrist structure 1500.

It has been discovered that the slope of the lower stop surfaces 1606 and the upper stop surfaces 1608 can be used to limit the amount of bend in the unitary wrist structure 1500 without further device or limiting mechanism. The slope of the lower stop surfaces 1606 and the upper stop surfaces 1608 thusly can eliminate external limiters, such as gear mechanism or limitation feature in software, and internal limiters, such as bumps or stoppers, and simplify manufacturing complexity and reduce manufacturing cost.

The lower relief clearance 1602 and the upper relief clearance 1604 can form a cylinder having a circular cross-section with the front side of the first pair of flexible hinges 1542. The lower relief clearance 1602 and the upper relief clearance 1604 can stretch to form a portion of a cylinder having an elliptical cross-section or a cross-section shaped similar to the numeral '3' on the backside of the first pair of flexible hinges 1542.

The material in the front side of the first pair of flexible hinges 1542 compresses when the first pair of flexible hinges 1542 bends forward. The material in the backside of the first pair of flexible hinges 1542 stretches when the first pair of flexible hinges 1542 bends forward.

The slopes of the abutting pair of the upper stop surface 1608 and the lower stop surface 1606 can limit the bend of the pairs of flexible hinges. For example, the magnitude of the angle away from a horizontal plane for the upper stop surface 1608 can be added with that of the lower stop surface 1606. The combined angle can be the maximum amount of bend for a given pair of flexible hinges.

Referring now to FIG. 19, therein is shown an enlarged side view of the unitary wrist structure partially flexed forward and partially flexed to the right. The unitary wrist structure 1500 is shown flexed forward as described above. The unitary wrist structure 1500 is shown also similarly flexed partially to the left.

The first connector portion 1532 has the same position relative to the second connector portion 1534 as shown in FIG. 18. The connector portions 1536-1540 of FIG. 15 have the same neutral position as shown in FIG. 16. The first connector portion 1532 and the second connector portion 1534 are shown rotated to the left.

The lower stop surface 1606 of FIG. 16 on the left side of the first connector portion 1532 can abut the upper stop surface 1608 of FIG. 16 on the left side of the second connector portion 1534. The first pair of flexible hinges 1542 can flex and bend to the left, similar to the second pair of flexible hinges 1544 as described above.

The flexible hinges 642-648 of FIG. 6, 1106 of FIG. 11, and 1542-1548 of FIG. 15 largely eliminate the pivot pin friction torques. The combined effect is to greatly reduce hysteresis manifested as lost or unpredictable motion in the unitary wrist structures 124 and 1500 as well as the unitary joint structure 1100.

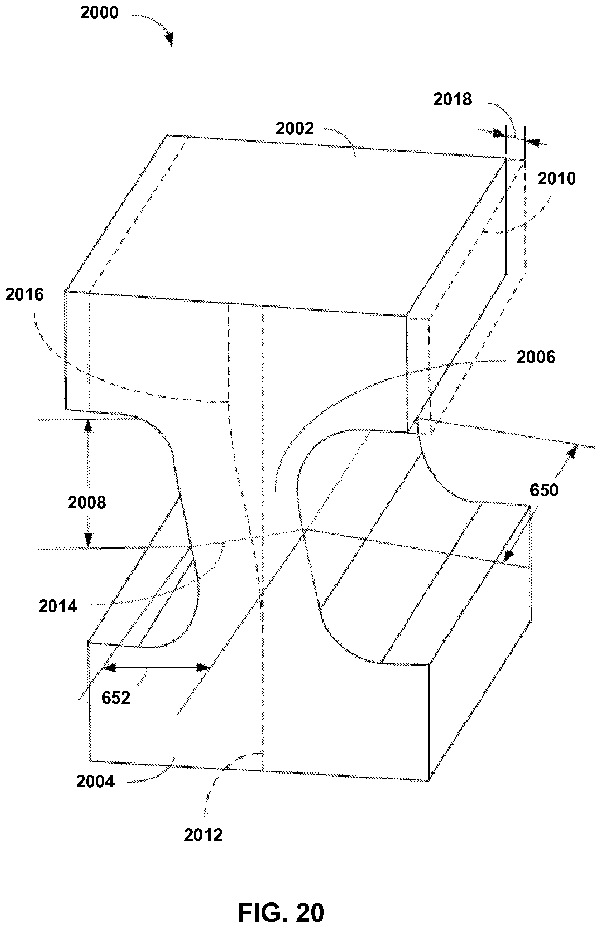

Referring now to FIG. 20, therein is shown an enlarged isometric view of a compact flexure structure 2000. The enlarged isometric view of the compact flexure structure 2000 depicts a first connection portion 2002 and a second connection portion 2004 having a compact flexure 2006 connected therebetween. A ghost line 2010 indicates the initial location of the first connection portion 2002, which has a resting centerline 2012, prior to the application of a transverse force or a torque, about the axis 624 of FIG. 6, the axis 1124 of FIG. 11, or axis 1524 of FIG. 15.

The compact flexure 2006 is a flexible hinge that has been designed to provide substantially equal shear and S-bending displacement or shear dominated displacement when the transverse force or the torque is applied to the application of the compact flexure 2006, such as the unitary wrist structure 124, of FIG. 1. A characteristic of the compact flexure 2006, when subjected to a transverse force or torque, is that the deflection due to an S-bend component 2016 is not more than two times the shear displacement caused by the transverse force or torque.

The compact flexure 2006 is characterized as having a beam length 2008, which represents half of the flexure length 654 of FIG. 6, and having the flexure thickness 652 and the flexure width 650. The measurement of the beam length 2008 from either the first connection portion 2002 or the second connection portion 2004 identifies a mid-plane 2014 of the compact flexure 2006. The mid-plane 2014 represents the position of the inflection point of an S-bend component 2016 of the deformation between the first connection portion 2002 or the second connection portion 2004 when either is moved transversely relative to the other.

The compact flexure 2006 is representative of the design principle of the flexible hinges 642-648 of FIG. 6, 1106 of FIG. 11, and 1542-1548 of FIG. 15 which are defined by opposing concave surfaces rather than flat surfaces. The dimensions of the beam length 2008 and the flexure thickness 652 are designed to provide substantially equal deflection in both shear and S-bending or less deflection in S-bending. The reduced contribution of the S-bending deflection is necessary in order to minimize the torsional and transverse compliance of the unitary wrist structure 124 of Fig. 1 of medical instrument 100 of Fig. 1 while permitting the intended bending of the wrist. A deflection distance is defined to be the sum of the distances that the compact flexure 2006 moves because of shear and S-bending when a transverse force is exerted on the compact flexure 2006, as shown in FIG. 20. The deflection distance may be calculated by the following equation:

$$\delta = \left(\frac{PL^3}{3EI}\right) + \left(\frac{PL}{kAG}\right) \qquad (1)$$

Where $\delta$ is half of the deflection 2018, which is the sum of the distance caused by bending and the distance caused by shear. In the bending deflection distance term:

P = load force applied to the member

L = the beam length 2008 as shown in FIG. 20.

E = Young's modulus

I = moment of inertia

In the shear deflection distance term:

P = the load force applied to the member

L = the beam length 2008 as shown in FIG. 20.

k = shear factor = 5/6 for rectangular beam cross-section

A = a cross-section area

G = shear modulus

Furthermore,

$$E = 2 * G \, ( \, 1 + v \, )$$

where $v$ = Poisson ratio

$$I = b * h^3 / 12$$

where b is the width 250 and h is the thickness 252 of the beam.

$$A = b * h$$

It will be understood by those skilled in the art that by equating the S-bending and shear deflection terms above and eliminating like factors the resulting simplified relation of flexure beam length to thickness is reached

$$L = h * \sqrt{(3(1+\nu)/5)} \qquad (2)$$

It has been discovered that because the length and thickness of compact flexure 1606 are proportioned to have the characteristic that the deflection due to S-bending is substantially equal to or less than the deflection due to shear, caused by a torque applied to the unitary wrist structure 124, of FIG. 6 about axis 624, unitary joint structure 1100, of FIG. 11 about axis 1124, or unitary wrist structure 1500 about axis 1524 will produce the S-bending that is greatly reduced. This balance between the S-bending displacement and the shear displacement minimizes the excessive rotational compliance and transverse compliance that are present in prior art flexible hinge wrists.

By way of an example, the compact flexure 2006 that is formed of polypropylene which has Poisson ratio ν = .42 can have equal shear and bending displacement contributions when the flexure beam length 2008 is equal to 0.92 times the flexure thickness 652. It is understood that the beam length 2008 is half of the overall flexure length 654, of FIG. 6. The compact flexure 2006 has the flexure length 654 that is less than twice the flexure thickness 652. The ratio of flexure length to thickness for the compact flexure 2006 with the desired reduced S-bending deflection substantially equivalent to or less than two times the shear deflection may vary somewhat for the unitary wrist and joint structures with concave flexure 2006 surfaces as in Figs 6 thru 19.

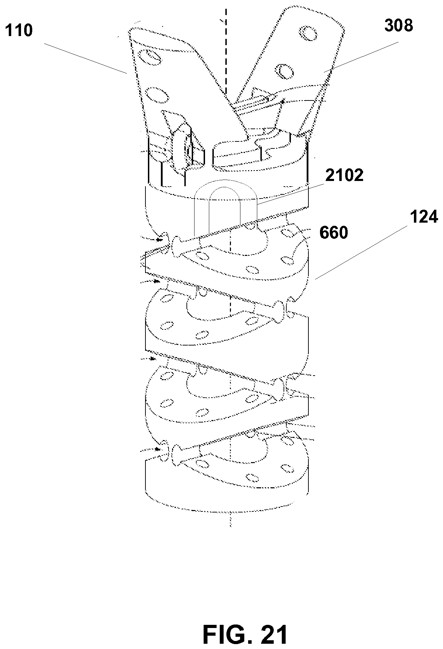

FIG. 21 illustrates unitary jaw structure 110 attached to unitary wrist structure 124. As discussed above, unitary jaw structure 110 can be formed by cutting or carving polypropylene plastic or metal alloy or can be formed by using wire electrical discharge machining (EDM) process and post treatment to remove surface layer re-melt, such as when used to shape Nitinol alloy. The unitary jaw structure 110 can be molded plastic or cast metal. The unitary jaw structure 110 can be formed as a single injection molding of a polymer, such as fiber reinforced polyether ether ketone (PEEK), or by metal injection molding (MIM) into a die or mold that has a continuous cavity. Flexible jaw mechanism 308 can also be formed by injection molding from plastic or fiber reinforced plastic composite, metal injection molding followed by sintering or by planar photo-lithographic metal plating. The jaw portion flexible hinge may be made of an alloy permitting high strains such as a hardened stainless steel, titanium or aluminum alloy or from Nitinol or other more advanced shape memory allow with even higher permissible strains or from metallic glass materials.

Unitary wrist structure 124 can also be carved or shaped out of a single unit of material, such as plastic or metal alloy. For example, unitary wrist structure 124 can be formed by cutting and carving polypropylene plastic or metal alloy or can be formed by using an EDM process and post treatment to remove surface layers re-melt, such as when used to shape Nitinol alloy. Unitary wrist structure 124 can also be molded plastic or cast or plated metal. The unitary wrist structure 124 can be formed as a single injection molding of polypropylene or by metal injection molding (MIM) into a die or mold that has a continuous cavity. As discussed above, immediately after removing unitary wrist structure 124 of polypropylene from the mold, the hinges 642-648 can be flexed to enhance flex life of unitary wrist structure 124.

As shown in FIG 21, unitary wrist structure 124 and unitary jaw structure 110 are joined. A passage 2102 allows actuating members 114 to pass from actuator holes 660 into unitary jaw mechanism 110. Passage 2102 may be formed internally or may be formed as grooves in the side of unitary jaw mechanism 110 to allow actuating members 114 to pass through pairs of actuator holes 660 as described above.

Unitary jaw structure 110 and unitary wrist structure 124 can be joined in several ways. For example, unitary jaw structure 110 and unitary wrist structure 124 can be formed from a single piece of material as described above. Unitary jaw structure 110 and unitary wrist structure 124 can be formed by injection molding or casting as a single piece as described above. Alternatively, unitary jaw mechanism 110 and unitary wrist structure 124 can be formed separately and combined, for example by forming a lip on unitary jaw structure 124 that receives a corresponding piece of unitary jaw mechanism 110 so that the two snap together. Further, unitary jaw mechanism 110 can be formed separately and unitary wrist mechanism formed by injection molding with unitary jaw mechanism 110 already in place, where unitary jaw mechanism includes structures around which is molded a portion of unitary wrist structure 124. Additionally, unitary jaw structure 110 can be bonded to unitary wrist structure 124.

The mechanisms of the present disclosure have been found to reduce the number of parts in flexible wrist mechanism to a single integrated part that can be fabricated in a few steps of machining, such as electrical discharge machining or other practical subtractive manufacturing processes.

It has been discovered that the unitary wrist structures 124 and 1500 as well as the unitary joint structure 1100 can also be fabricated as a single piece by additive processes such as injection molding from plastic or fiber reinforced plastic composite, metal injection molding followed by sintering or by planar photo-lithographic metal plating thus reducing the part cost and the assembly time. The flexible hinge may be made of an alloy permitting high strains such as a hardened stainless steel, titanium or aluminum alloy or from Nitinol or other more advanced shape memory alloy with even higher permissible strains or from metallic glass materials.

The resulting method, process, apparatus, device, product, and/or system is straightforward, cost-effective, uncomplicated, highly versatile, accurate, sensitive, and effective, and can be implemented by adapting known materials and processes for ready, efficient, and economical manufacturing, application, and utilization.

Another important aspect of the present invention is that it valuably supports and services the historical trend of reducing costs, simplifying systems, and increasing performance.

These and other valuable aspects of the present invention consequently further the state of the technology to at least the next level.

While the invention has been described in conjunction with a specific best mode, it is to be understood that many alternatives, modifications, and variations will be apparent to those skilled in the art in light of the aforegoing description. Accordingly, it is intended to embrace all such alternatives, modifications, and variations that fall within the scope of the included claims. All matters hithertofore set forth herein or shown in the accompanying drawings are to be interpreted in an illustrative and non-limiting sense.

**Claims**

1. A medical instrument comprising:
   a unitary jaw structure (110) having:

   a connector portion (206) with a width,
   a first jaw portion (202) flexibly integral with the connector portion (206) at one side of the width,
   a second jaw portion (208) integral with the connector portion (206) at the other side of the width,
   a first arm portion (302) integral with the first jaw portion (202) and extending from the first jaw portion (202) to the other side of the width, and
   an actuator portion (204) flexibly integral with the first arm portion (302) at the other side of the width for causing rotating motion of the first jaw portion (202) upon linear motion of the actuator portion (204), the actuator portion (204) extending centrally through the connector portion (206),
   wherein the connector portion (206), the first jaw portion (202), the second jaw portion (208), the first arm portion (302), and the actuator portion (204) are all formed in a single piece.

2. The instrument as claimed in claim 1 wherein the unitary jaw structure (110) includes compact flexure hinges formed into the single piece.

3. The instrument as claimed in claim 1 wherein the first jaw portion (202) includes an adaptor for accommodating a task adaptor (220) of different shapes and different materials for different uses of the unitary jaw structure (110).

4. The instrument as claimed in claim 1 wherein
   the unitary jaw structure (110) further including a second arm portion (402) flexibly integral with the second jaw portion (208) and the actuator portion (204) for causing rotating motion of the second jaw portion (208) upon the linear motion of the actuator portion (204); and
   the second arm portion (402) being formed integrally with the connector portion (206), the first jaw portion (202), the second jaw portion (208), the first arm portion (302), and actuator portion (204) .

5. The instrument as claimed in claim 1 wherein the unitary jaw structure (110) has a taper shape narrower at a distal end.

6. The instrument as claimed in claim 1 wherein the unitary jaw structure (110) has a cylindrical configuration.

7. The instrument as claimed in claim 1 wherein the unitary jaw structure (110) is made of a moldable, shapeable, or joinable plastic or metal.

8. The instrument as claimed in claim 1 further comprising a wrist mechanism connected to the connector portion (206) for orienting the unitary jaw structure (110).

9. The instrument as claimed in claim 1 further comprising:

a tube connected directly to the connector portion (206) for holding the connector portion (206) stationary relative to the motion of the actuator portion (204); and

actuating members coupled to the actuator portion (204)for causing reciprocating motion of the actuator portion (204).

10. The instrument as claimed in claim 1, further including a force sensor at the actuator portion (204).

11. The instrument of claim 1, wherein the force sensor is based on measurement of an electrical current applied to a motor or pressure applied to a hydraulic or pneumatic actuator.

12. The medical instrument of claim 1, wherein the:

unitary jaw structure (110) further comprises:

a first flexible hinge (216) integral with the connector portion (206) at one side of the width, the first jaw portion (202) being integral with the first flexible hinge (216), and a first arm hinge portion (304) integral with the first arm portion (302), the actuator portion (204) being integral with the first arm hinge portion (304).

## Patentansprüche

1. Medizinisches Instrument, das Folgendes umfasst:
eine einheitliche Backenstruktur (110) mit:

einem Verbinderabschnitt (206) mit einer Breite,
einem ersten Backenabschnitt (202), der mit dem Verbinderabschnitt (206) auf einer Seite der Breite flexibel integriert ist,
einem zweiten Backenabschnitt (208), der mit dem Verbinderabschnitt (206) auf der anderen Seite der Breite integriert ist,
einem ersten Armabschnitt (302), der mit dem ersten Backenabschnitt (202) integriert ist und vom ersten Backenabschnitt (202) zur anderen Seite der Breite verläuft, und
einem Aktuatorabschnitt (204), der mit dem ersten Armabschnitt (302) auf der anderen Seite der Breite flexibel integriert ist, um eine Rotationsbewegung des ersten Backenabschnitts (202) nach einer Linearbewegung des Aktuatorabschnitts (204) zu bewirken, wobei der Aktuatorabschnitt (204) zentral durch den Verbinderabschnitt (206) verläuft,
wobei der Verbinderabschnitt (206), der erste Backenabschnitt (202), der zweite Backenabschnitt (208), der erste Armabschnitt (302) und der Aktuatorabschnitt (204) alle in einem Stück ausgebildet sind.

2. Instrument nach Anspruch 1, wobei die einheitliche Backenstruktur (110) kompakte Festkörpergelenke beinhaltet, die zu dem einzigen Stück ausgebildet sind.

3. Instrument nach Anspruch 1, wobei der erste Backenabschnitt (202) einen Adapter zum Aufnehmen eines Aufgabenadapters (220) von unterschiedlichen Formen und aus unterschiedlichen Materialien für unterschiedliche Verwendungszwecke der einheitlichen Backenstruktur (110) beinhaltet.

4. Instrument nach Anspruch 1, wobei
die einheitliche Backenstruktur (110) ferner einen zweiten Armabschnitt (402) aufweist, der mit dem zweiten Backenabschnitt (208) und dem Aktuatorabschnitt (204) flexibel integriert ist, um eine Rotationsbewegung des zweiten Backenabschnitts (208) nach der Linearbewegung des Aktuatorabschnitts (204) zu bewirken; und
der zweite Armabschnitt (402) mit dem Verbinderabschnitt (206), dem ersten Backenabschnitt (202), dem zweiten Backenabschnitt (208), dem ersten Armabschnitt (302) und dem Aktuatorabschnitt (204) integriert ausgebildet ist.

5. Instrument nach Anspruch 1, wobei die einheitliche Backenstruktur (110) eine Kegelform hat, die an einem distalen Ende schmäler ist.

6. Instrument nach Anspruch 1, wobei die einheitliche Backenstruktur (110) eine zylindrische Konfiguration hat.

7. Instrument nach Anspruch 1, wobei die einheitliche Backenstruktur (110) aus einem formbaren, gestaltbaren oder zusammenfügbaren Plastik oder Metall ist.

8. Instrument nach Anspruch 1, das ferner einen Gelenkmechanismus umfasst, der mit dem Verbinderabschnitt (206) verbunden ist, um die einheitliche Backenstruktur (110) zu orientieren.

9. Instrument nach Anspruch 1, das ferner Folgendes beinhaltet:

ein Rohr, das direkt mit dem Verbinderabschnitt (206) verbunden ist, um den Verbinderabschnitt (206) relativ zur Bewegung des Aktuatorabschnitts (204) stationär zu halten; und
Betätigungselemente, die mit dem Aktuatorabschnitt (204) gekoppelt sind, um eine Hin- und Herbewegung des Aktuatorabschnitts (204) zu bewirken.

10. Instrument nach Anspruch 1, das ferner einen Kraftsensor am Aktuatorabschnitt (204) aufweist.

11. Instrument nach Anspruch 1, wobei der Kraftsensor auf einer Messung eines einem Motor zugeführten elektrischen Stroms oder eines auf einen hydraulischen oder pneumatischen Aktuator aufgebrachten Drucks basiert.

12. Instrument nach Anspruch 1, wobei
die einheitiche Backenstruktur (110) ferner Folgendes umfasst:

ein erstes flexibles Gelenk (216), das mit dem Verbinderabschnitt (206) auf einer Seite der Breite integriert ist, wobei der erste Backabschnitt (202) mit dem ersten flexiblen Gelenk (216) integriert ist, und
einen ersten Armgelenkabschnitt (304) integriert mit dem ersten Armabschnitt (302), wobei der Aktuatorabschnitt (204) mit dem ersten Armgelenkabschnitt (304) integriert ist.

## Revendications

1. Instrument médical comprenant :
une structure de mâchoire unitaire (110) comportant :

une partie formant un connecteur (206) ayant une largeur,
une première partie de mâchoire (202) formant une seule pièce de façon flexible avec la partie formant un connecteur (206) d'un côté de la largeur,
une deuxième partie de mâchoire (208) formant une seule pièce avec la partie formant un connecteur (206) de l'autre côté de la largeur,
une première partie formant un bras (302) formant une seule pièce avec la première partie de mâchoire (202) et s'étendant de la première partie de mâchoire (202) à l'autre côté de la largeur, et
une partie formant un actionneur (204) formant une seule pièce de façon flexible avec la première partie formant un bras (302) de l'autre côté de la largeur pour provoquer un déplacement en rotation de la première partie de mâchoire (202) lors d'un déplacement linéaire de la partie formant un actionneur (204), la partie formant un actionneur (204) s'étendant centralement à travers la partie formant un connecteur (206),
dans lequel la partie formant un connecteur (206), la première partie de mâchoire (202), la deuxième partie de mâchoire (208), la première partie formant un bras (302) et la partie formant un actionneur (204) sont toutes formées en une seule pièce.

2. Instrument selon la revendication 1, dans lequel la structure de mâchoire unitaire (110) comprend des charnières d'articulation compactes formées en ladite une seule pièce.

3. Instrument selon la revendication 1, dans lequel la première partie de mâchoire (202) comprend un adaptateur pour recevoir un adaptateur de tâche (220) de différentes formes et de différents matériaux pour différentes utilisations de la structure de mâchoire unitaire (110).

4. Instrument selon la revendication 1, dans lequel
la structure de mâchoire unitaire (110) comprend en outre une deuxième partie formant un bras (402) formant une seule pièce de façon flexible avec la deuxième partie de mâchoire (208) et la partie formant un actionneur (204) pour provoquer un déplacement en rotation de la deuxième partie de mâchoire (208) lors du déplacement linéaire de la partie formant un actionneur (204) ; et
la deuxième partie formant un bras (402) formant une seule pièce avec la partie formant un connecteur (206), la première partie de mâchoire (202), la deuxième partie de mâchoire (208), la première partie formant un bras (302), et la partie formant un actionneur (204).

5. Instrument selon la revendication 1, dans lequel la structure de mâchoire unitaire (110) a une forme conique plus étroite à une extrémité distale.

6. Instrument selon la revendication 1, dans lequel la structure de mâchoire unitaire (110) a une configuration cylindrique.

7. Instrument selon la revendication 1, dans lequel la structure de mâchoire unitaire (110) se compose d'un plastique ou d'un métal capable d'être moulé, mis en forme ou assemblé.

8. Instrument selon la revendication 1, comprenant en outre un mécanisme de poignet raccordé à la partie formant un connecteur (206) pour orienter la structure de mâchoire unitaire (110).

9. Instrument selon la revendication 1, comprenant en outre :

   un tube raccordé directement à la partie formant un connecteur (206) pour maintenir la partie formant un connecteur (206) stationnaire relativement au déplacement de la partie formant un actionneur (204) ; et
   des éléments d'actionnement couplés à la partie formant un actionneur (204) pour provoquer un déplacement alternatif de la partie formant un actionneur (204).

10. Instrument selon la revendication 1, comprenant en outre un capteur de force au niveau de la partie formant un actionneur (204).

11. Instrument selon la revendication 1, dans lequel le capteur de force est basé sur une mesure d'un courant électrique appliqué à un moteur ou d'une pression appliquée à un actionneur hydraulique ou pneumatique.

12. Instrument médical selon la revendication 1, dans lequel la structure de mâchoire unitaire (110) comprend en outre :

   une première charnière d'articulation flexible (216) formant une seule pièce avec la partie formant un connecteur (206) d'un côté de la largeur,
   la première partie de mâchoire (202) formant une seule pièce avec la première charnière d'articulation flexible (216), et
   une première partie formant une charnière d'articulation de bras (304) formant une seule pièce avec la première partie formant un bras (302), la partie formant un actionneur (204) formant une seule pièce avec la première partie formant une charnière d'articulation de bras (304).

FIG. 1

FIG. 2

FIG. 3

FIG. 4

FIG. 5

**FIG. 6**

**FIG. 7**

**FIG. 8**

**FIG. 9**

**FIG. 10**

1100

1112
1102

1110
1108
1104

1106

1124

**FIG. 11**

1100

1102

1202
1106
1204

1104

**FIG. 12**

1100

1100
1304

1302
1106
1102

1104

**FIG. 13**

1100

1302

1304

**FIG. 14**

FIG. 15

**FIG. 16**

**FIG. 17**

**FIG. 18**

**FIG. 19**

**FIG. 20**

FIG. 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 19537320

- US 6817974 B